(19) Europäisches Patentamt
European Patent Office
Office européen des brevets

(11) EP 3 393 438 B1

(12) # EUROPEAN PATENT SPECIFICATION

(45) Date of publication and mention of the grant of the patent:
**29.01.2020 Bulletin 2020/05**

(21) Application number: **16809435.7**

(22) Date of filing: **15.12.2016**

(51) Int Cl.:
***A61K 8/39*** (2006.01) ***A61K 8/41*** (2006.01)
***A61Q 5/06*** (2006.01) ***A61K 8/81*** (2006.01)
***A61K 8/898*** (2006.01) ***A61K 8/04*** (2006.01)

(86) International application number:
**PCT/EP2016/081152**

(87) International publication number:
**WO 2017/108565 (29.06.2017 Gazette 2017/26)**

(54) **COMPOSITION COMPRISING A VINYLFORMAMIDE/VINYLAMINE COPOLYMER, A FIXING POLYMER AND A PARTICULAR COMBINATION OF SURFACTANTS**

ZUSAMMENSETZUNG MIT EINEM VINYLFORMAMID-/VINYLAMINCOPOLYMER, FIXIERUNGSPOLYMER UND EINE BESONDERE KOMBINATION AUS TENSIDEN

COMPOSITION COMPRENANT UN COPOLYMÈRE VINYLFORMAMIDE/VINYLAMINE, UN POLYMÈRE DE FIXATION ET UNE COMBINAISON PARTICULIÈRE DE TENSIOACTIFS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.12.2015 FR 1562944**

(43) Date of publication of application:
**31.10.2018 Bulletin 2018/44**

(73) Proprietor: **L'Oréal**
**75008 Paris (FR)**

(72) Inventors:
- **PASQUET, Dorothée**
**93400 Saint Ouen (FR)**
- **DA SILVA, Sylvia**
**93400 Saint Ouen (FR)**

(74) Representative: **Casalonga**
**Casalonga & Partners**
**Bayerstraße 71/73**
**80335 München (DE)**

(56) References cited:
**EP-A1- 1 205 174**
**EP-A2- 1 169 998**
**EP-A2- 2 098 217**
**WO-A1-2009/103735**
**WO-A2-2013/083350**
**FR-A1- 2 926 990**
**FR-A1- 3 002 142**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

The invention relates to a composition, in particular a cosmetic composition, comprising at least one vinylformamide/vinylamine copolymer, at least one fixing polymer and a combination of surfactants comprising at least one particular nonionic surfactant and at least one cationic surfactant.

The present invention also relates to a cosmetic process for treating the hair, especially a process for fixing and/or shaping the hair using this composition.

Finally, the present invention relates to the use of this composition for cosmetic hair treatment, and in particular for hair styling, i.e. shaping and/or fixing the hairstyle.

Styling products are normally used to construct and structure the hairstyle and to give it shape retention. They are usually in the form of lotions, gels, mousses, creams, sprays, etc. These compositions generally comprise one or more film-forming polymers or "fixing polymers". These polymers allow the formation of a coating film on the hair, thus providing form retention of the hairstyle.

These compositions may be in the form of hair gels or foams (mousses) which are generally applied to wet hair, which is shaped before performing blow drying or drying.

To obtain satisfactory and long-lasting fixing power, it is known practice to incorporate into styling products polymers with very high fixing power, and/or to increase the concentration of fixing polymer. However, the use of such extremely fixing products causes a certain number of drawbacks. In particular, these products may result in the hair having a dry and rough feel.

Furthermore, although these products make it possible to fix and hold the hairstyle over time, they nevertheless have a tendency to make the hairstyle rigid, in particular producing a "helmet effect". This effect is often poorly perceived by users.

Such products are disclosed in the prior art, for instance in patent applications FR 3 002 142 and EP 2 098 217.

Moreover, when products in mousse form are used, they may have more or less firm textures with limited hold in the hand.

There is thus a need for hair compositions that can produce long-lasting fixing of the hairstyle, with styling effects that last throughout the day or even for several days, while at the same time giving the hairstyle a natural, non-rigid look, and affording a pleasant cosmetic feel, especially a soft, smooth feel.

The Applicant has now discovered that a composition comprising one or more vinylformamide/vinylamine copolymers, one or more fixing polymers, one or more particular nonionic surfactants and one or more cationic surfactants makes it possible to solve the problems mentioned above.

One subject of the invention is thus a composition comprising one or more vinylformamide/vinylamine copolymers, one or more fixing polymers other than vinylformamide/vinylamine copolymers, one or more nonionic surfactants chosen from oxyalkylenated, preferably oxyethylenated, fatty alcohols, and one or more cationic surfactants.

The composition according to the invention especially affords good hairstyle hold over time under both wet and dry conditions, while at the same time giving the head of hair volume, and without making the hairstyle rigid. In the case of curly hair, the composition according to the invention affords good curl definition.

In particular, the composition according to the invention gives the hairstyle a natural look and fluid movement.

It also gives the hair a particularly soft and pleasant feel.

According to a preferred embodiment, the composition according to the invention also contains one or more propellants.

In this embodiment, the composition according to the invention makes it possible to form a homogeneous, firm and creamy foam, which holds up well in the hand.

Furthermore, the foam formed using such a composition distributes easily and uniformly over the hair, and has good styling properties.

A subject of the invention is also the use of the composition according to the invention for styling and shaping the hair.

A subject of the invention is also a process for shaping keratin fibres, preferably the hair, comprising the application to said fibres of the composition according to the invention.

Other subjects, characteristics, aspects and advantages of the invention will emerge even more clearly on reading the description and the example that follows.

In that which follows and unless otherwise indicated, the limits of a range of values are included within this range, in particular in the expressions "of between" and "ranging from ... to ...".

Moreover, the expression "at least one" used in the present description is equivalent to the expression "one or more".

The vinylformamide/vinylamine copolymers used in the composition according to the invention comprise:

- vinylamine units of formula (IA):

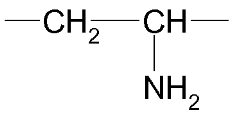 (IA),

and

- vinylformamide units of formula (IB):

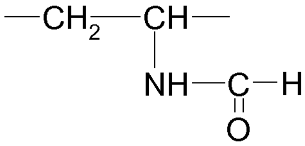 (IB).

Preferably, these copolymers are chosen from polymers comprising from 10 mol% to 95 mol% of units of formula (IA) and from 90 mol% to 5 mol% of units of formula (IB).

The vinylformamide/vinylamine copolymer(s) preferably comprise from 10 mol% to 60 mol% of units of formula (IA) and more particularly from 20 mol% to 40 mol%.

The vinylformamide/vinylamine copolymer(s) preferably comprise from 30 mol% to 90 mol% of units of formula (IB) and more particularly from 60 mol% to 80 mol%.

These copolymers may be obtained, for example, by partial hydrolysis of polyvinylformamide. This hydrolysis may take place in acidic or basic medium.

The vinylformamide/vinylamine copolymer(s) according to the invention may optionally comprise one or more additional monomer units. In this case, the latter preferably represent(s) less than 20 mol% of the copolymer.

According to a preferred embodiment, the vinylformamide/vinylamine copolymer(s) according to the invention are formed solely from units of formula (IA) and units of formula (IB).

The weight-average molecular mass of the copolymer, measured by light scattering, may range from 10 000 to 30 000 000 g/mol, preferably from 40 000 to 1 000 000 g/mol and more particularly from 100 000 to 500 000 g/mol.

The cationic charge density of the vinylformamide/vinylamine copolymer may range from 2 meq/g to 20 meq/g, preferably from 2.5 to 15 meq/g and more particularly from 3.5 to 10 meq/g.

As example of vinylformamide/vinylamine copolymers that may be used in the compositions according to the invention, mention is made, inter alia, of the products sold under the names Luviquat and Lupamin by the company BASF, for instance, and without limitation, the products sold under the names Luviquat 9030, Lupamin 9095, Lupamin 5095, Lupamin 1095, Lupamin 9030 and Lupamin 9010.

The vinylformamide/vinylamine copolymer(s) are present in the compositions according to the invention in proportions preferably ranging from 0.01% to 20% by weight, more preferentially from 0.1% to 10% by weight and more particularly from 0.15% to 5% by weight, relative to the total weight of the composition.

The composition according to the invention comprises one or more nonionic surfactants chosen from oxyalkylenated fatty alcohols. These surfactants are more preferentially chosen from oxyethylenated fatty alcohols.

For the purposes of the present invention, the term "fatty alcohol" means an alcohol comprising a linear or branched, saturated or unsaturated alkyl chain, comprising at least 8 carbon atoms.

Preferably, such a fatty alcohol comprises from 8 to 30 carbon atoms, more preferably from 10 to 24 carbon atoms and better still from 12 to 22 carbon atoms.

Preferably, the fatty alcohol according to the invention comprises a saturated linear alkyl chain.

The number of oxyalkylene units, preferably oxyethylene units, preferably ranges from 1 to 50, more preferentially from 2 to 20, better still from 3 to 10 and even better still from 3 to 6.

The oxyalkylenated fatty alcohols that are particularly preferred are chosen from oxyethylenated fatty alcohols comprising a C12-C22 alkyl chain and from 3 to 10 oxyethylene units, better still chosen from oxyethylenated fatty alcohols comprising a C12-C22 alkyl chain and from 3 to 6 oxyethylene units.

A particularly preferred compound corresponds to the INCI name Laureth-4 (lauryl alcohol containing 4 oxyethylene units).

The content in the composition according to the invention of nonionic surfactants chosen from oxyalkylenated, preferably oxyethylenated, fatty alcohols preferably ranges from 0.01% to 5% by weight, more preferentially from 0.05% to 2% by weight and even more preferentially from 0.1% to 1% by weight, relative to the total weight of the composition.

As stated previously, the composition according to the invention comprises one or more cationic surfactants.

The term "cationic surfactant" means a surfactant that is positively charged when it is contained in the compositions according to the invention. This surfactant may bear one or more positive permanent charges or may contain one or more cationizable functions in the compositions according to the invention.

The cationic surfactant(s) are preferably chosen from primary, secondary or tertiary fatty amines, which are optionally polyoxyalkylenated, or salts thereof, and quaternary ammonium salts, and mixtures thereof.

The fatty amines generally comprise at least one $C_8$ to $C_{30}$ hydrocarbon-based chain.

Mention may in particular be made, as quaternary ammonium salts, for example, of:

- those corresponding to the general formula (II) below:

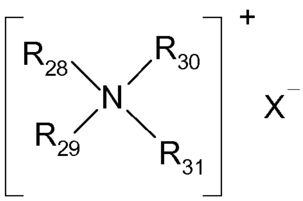

(II)

in which the groups $R_{28}$ to $R_{31}$, which may be identical or different, represent a linear or branched aliphatic group containing from 1 to 30 carbon atoms, or an aromatic group such as aryl or alkylaryl, at least one of the groups $R_{28}$ to $R_{31}$ denoting a group containing from 8 to 30 carbon atoms, preferably from 12 to 24 carbon atoms. The aliphatic groups may comprise heteroatoms such as, in particular, oxygen, nitrogen, sulfur and halogens. The aliphatic groups are chosen, for example, from $C_1$-$C_{30}$ alkyl, $C_1$-$C_{30}$ alkoxy, polyoxy($C_2$-$C_6$)alkylene, $C_1$-$C_{30}$ alkylamide, ($C_{12}$-$C_{22}$)alkylamido($C_2$-$C_6$)alkyl, ($C_{12}$-$C_{22}$)alkyl acetate, and $C_1$-$C_{30}$ hydroxyalkyl groups, $X^-$ is an anion chosen from the group of halides, phosphates, acetates, lactates, ($C_1$-$C_4$)alkyl sulfates, and ($C_1$-$C_4$)alkyl- or ($C_1$-$C_4$)alkylarylsulfonates.

Among the quaternary ammonium salts of formula (II), those that are preferred are, on the one hand, tetraalkylammonium salts, for instance dialkyldimethylammonium or alkyltrimethylammonium salts in which the alkyl group contains approximately from 12 to 22 carbon atoms, in particular behenyltrimethylammonium, distearyldimethylammonium, cetyltrimethylammonium or benzyldimethylstearylammonium salts, or, on the other hand, the palmitylamidopropyltrimethylammonium salts, the stearamidopropyltrimethylammonium salts, the stearamidopropyldimethylcetearylammonium salts, or the stearamidopropyldimethyl(myristyl acetate)ammonium salts sold under the name Ceraphyl® 70 by the company Van Dyk. It is preferable in particular to use the chloride salts of these compounds.

- quaternary ammonium salts of imidazoline, for instance those of formula (III) below:

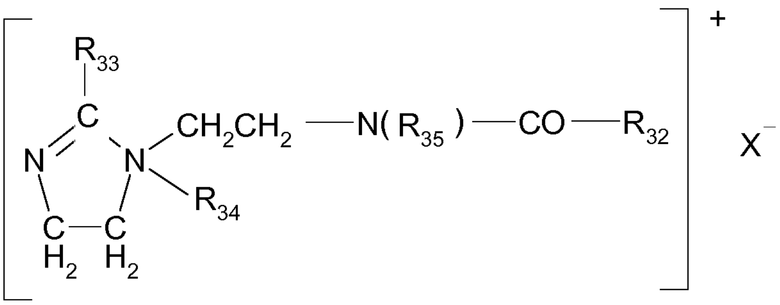

(III)

in which:

- $R_{32}$ represents an alkenyl or alkyl group comprising from 8 to 30 carbon atoms, for example derived from tallow fatty acids,
- $R_{33}$ represents a hydrogen atom, a $C_1$ to $C_4$ alkyl group or an alkenyl or alkyl group comprising from 8 to 30 carbon atoms,
- $R_{34}$ represents a $C_1$ to $C_4$ alkyl group,
- $R_{35}$ represents a hydrogen atom or a $C_1$ to $C_4$ alkyl group,
- $X^-$ is an anion chosen from the group of halides, phosphates, acetates, lactates, alkyl sulfates, alkyl- or alkylaryl-sulfonates in which the alkyl and aryl groups preferably comprise, respectively, from 1 to 20 carbon atoms and from 6 to 30 carbon atoms.

Preferably, $R_{32}$ and $R_{33}$ denote a mixture of alkenyl or alkyl groups comprising from 12 to 21 carbon atoms, for example derived from tallow fatty acids, $R_{34}$ denotes a methyl group and $R_{35}$ denotes a hydrogen atom. Such a product is sold, for example, under the name Rewoquat® W 75 by Rewo.

- quaternary di- or triammonium salts in particular of formula (IV):

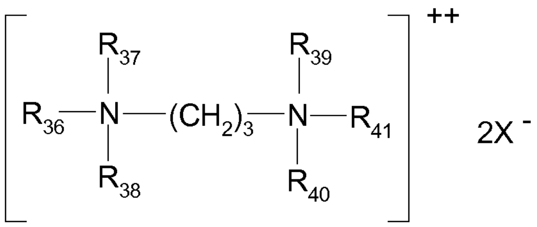

(IV)

in which:

- $R_{36}$ denotes an alkyl radical comprising approximately from 16 to 30 carbon atoms, which is optionally hydroxylated and/or interrupted with one or more oxygen atoms,
- $R_{37}$ is chosen from hydrogen, an alkyl radical comprising from 1 to 4 carbon atoms or a group $(R_{36a})(R_{37a})(R_{38a})N\text{-}(CH_2)_3$,
- $R_{36a}$, $R_{37a}$, $R_{38a}$, $R_{38}$, $R_{39}$, $R_{40}$ and $R_{41}$, which may be identical or different, are chosen from hydrogen and an alkyl radical comprising from 1 to 4 carbon atoms, and
- $X^-$ is an anion chosen from the group of halides, acetates, phosphates, nitrates and methyl sulfates.

Such compounds are, for example, Finquat CT-P available from the company Finetex (Quaternium 89), and Finquat CT, available from the company Finetex (Quaternium 75).

- quaternary ammonium salts containing at least one ester function, such as those of formula (V) below:

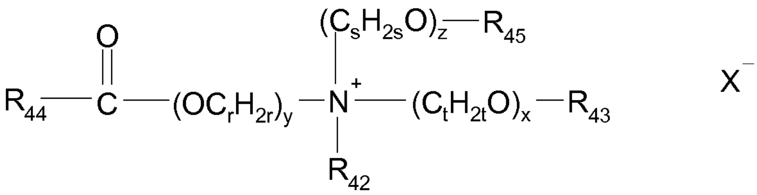

(V)

in which:

- $R_{42}$ is chosen from $C_1$ to $C_6$ alkyl groups and $C_1$ to $C_6$ hydroxyalkyl or dihydroxyalkyl groups;
- $R_{43}$ is chosen from:
    - the group

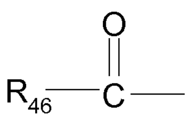

    - groups $R_{47}$, which are saturated or unsaturated and linear or branched $C_1$ to $C_{22}$ hydrocarbon-based groups,
    - a hydrogen atom,

- $R_{45}$ is chosen from:

- the group

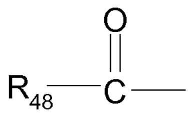

- groups $R_{49}$, which are saturated or unsaturated and linear or branched $C_1$ to $C_6$ hydrocarbon-based groups,
- a hydrogen atom,

- $R_{44}$, $R_{46}$ and $R_{48}$, which may be identical or different, are chosen from linear or branched, saturated or unsaturated $C_7$ to $C_{21}$ hydrocarbon-based groups,
- r, s and t, which may be identical or different, are integers having values from 2 to 6;
- y is an integer having a value from 1 to 10;
- x and z, which may be identical or different, are integers having a value from 0 to 10;
- $X^-$ is a simple or complex and organic or mineral anion;

with the proviso that the sum x + y + z is from 1 to 15, that when x is 0 then $R_{43}$ denotes $R_{47}$, and that when z is 0 then $R_{45}$ denotes $R_{49}$.

The alkyl groups $R_{42}$ may be linear or branched, and more particularly linear.

Preferably, $R_{42}$ denotes a methyl, ethyl, hydroxyethyl or dihydroxypropyl group, and more particularly a methyl or ethyl group.

Advantageously, the sum x + y + z is from 1 to 10.

When $R_{43}$ is a hydrocarbon-based group $R_{47}$, it may be long and contain from 12 to 22 carbon atoms, or may be short and contain from 1 to 3 carbon atoms.

When $R_{45}$ is a hydrocarbon-based group $R_{49}$, it preferably contains 1 to 3 carbon atoms.

Advantageously, $R_{44}$, $R_{46}$ and $R_{48}$, which may be identical or different, are chosen from linear or branched, saturated or unsaturated $C_{11}$ to $C_{21}$ hydrocarbon-based groups, and more particularly from linear or branched, saturated or unsaturated $C_{11}$ to $C_{21}$ alkyl and alkenyl groups.

Preferably, x and z, which may be identical or different, have the value 0 or 1.

Advantageously, y is equal to 1.

Preferably, r, s and t, which may be identical or different, have the value 2 or 3 and more particularly still are equal to 2.

The anion $X^-$ is preferably a halide (chloride, bromide or iodide) or an alkyl sulfate, more particularly methyl sulfate. However, use may be made of methanesulfonate, phosphate, nitrate, tosylate, an anion derived from an organic acid, such as acetate or lactate, or any other anion which is compatible with the ammonium having an ester function.

The anion $X^-$ is more particularly still chloride or methyl sulfate.

Use is made more particularly in the composition according to the invention of the ammonium salts of formula (V) in which:

$R_{42}$ denotes a methyl or ethyl group,
x and y are equal to 1;
z is equal to 0 or 1;
r, s and t are equal to 2;
$R_{43}$ is chosen from:

- the group

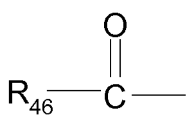

- methyl, ethyl or $C_{14}$ to $C_{22}$ hydrocarbon-based groups,
- a hydrogen atom;

$R_{45}$ is chosen from:

- the group

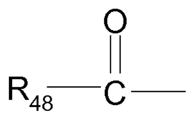

- a hydrogen atom;

$R_{44}$, $R_{46}$ and $R_{48}$, which may be identical or different, are chosen from saturated or unsaturated and linear or branched $C_{13}$ to $C_{17}$ hydrocarbon-based groups and preferably from saturated or unsaturated and linear or branched $C_{13}$ to $C_{17}$ alkyl and alkenyl groups.

Advantageously, the hydrocarbon-based groups are linear.

Examples that may be mentioned include the compounds having formula (V) such as the diacyloxyethyldimethylammonium, diacyloxyethylhydroxyethylmethylammonium, monoacyloxyethyldihydroxyethylmethylammonium, triacyloxyethylmethylammonium and monoacyloxyethylhydroxyethyldimethylammonium salts (chloride or methyl sulfate in particular), and mixtures thereof. The acyl groups preferably contain 14 to 18 carbon atoms and are obtained more particularly from a plant oil such as palm oil or sunflower oil. When the compound contains several acyl groups, these groups may be identical or different.

These products are obtained, for example, by direct esterification of triethanolamine, triisopropanolamine, alkyldiethanolamine or alkyldiisopropanolamine, which are optionally oxyalkylenated, with $C_{10}$ to $C_{30}$ fatty acids or with mixtures of $C_{10}$ to $C_{30}$ fatty acids of vegetable or animal origin, or by transesterification of their methyl esters. This esterification is followed by a quaternization using an alkylating agent such as an alkyl halide (preferably a methyl or ethyl halide), a dialkyl sulfate (preferably a dimethyl or diethyl sulfate), methyl methanesulfonate, methyl para-toluenesulfonate, glycol chlorohydrin or glycerol chlorohydrin.

Such compounds are sold, for example, under the names Dehyquart® by the company Henkel, Stepanquat® by the company Stepan, Noxamium® by the company CECA or Rewoquat® WE 18 by the company Rewo-Witco.

The composition according to the invention may contain, for example, a mixture of quaternary ammonium monoester, diester and triester salts with a majority by weight of diester salts.

It is also possible to use the ammonium salts containing at least one ester function that are described in patents US-A-4 874 554 and US-A-4 137 180.

Use may be made of behenoylhydroxypropyltrimethylammonium chloride sold by KAO under the name Quatarmin BTC 131.

Preferably, the ammonium salts comprising at least one ester function comprise two ester functions.

Among the quaternary ammonium salts containing at least one ester function, which may be used, it is preferred to use dipalmitoylethylhydroxyethylmethylammonium salts.

The cationic surfactants are preferably chosen from those of formula (II) and those of formula (V) and even more preferentially from those of formula (II).

The content of cationic surfactant(s) in the composition according to the invention preferably ranges from 0.01% to 10% by weight, more preferentially from 0.02% to 5% by weight and better still from 0.05% to 2% by weight relative to the total weight of the composition.

As stated previously, the composition according to the invention comprises one or more fixing polymers, other than the vinylformamide/vinylamine copolymers described above.

The term "fixing polymer" means any polymer that is capable of giving a shape to a head of hair or of holding a head of hair in a given shape.

The fixing polymer(s) used may be chosen from anionic, cationic, amphoteric and nonionic fixing polymers, and mixtures thereof.

Anionic fixing polymers that may be mentioned include polymers containing groups derived from carboxylic, sulfonic or phosphoric acids, and having a number-average molecular mass of between 500 and 5 000 000.

The carboxylic groups are provided by unsaturated monocarboxylic or dicarboxylic acid monomers, such as those corresponding to the formula:

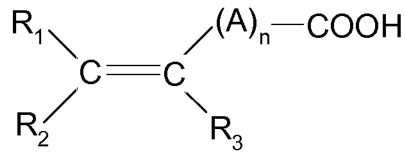

(VI)

in which n is an integer from 0 to 10, A denotes a methylene group which is optionally connected to the carbon atom of the unsaturated group or to the neighbouring methylene group when n is greater than 1, via a heteroatom such as oxygen or sulfur, $R_1$ denotes a hydrogen atom or a phenyl or benzyl group, $R_2$ denotes a hydrogen atom, an alkyl group containing from 1 to 4 carbon atoms, or a carboxyl group, $R_3$ denotes a hydrogen atom, an alkyl group containing 1 to 4 carbon atoms, or a $-CH_2-COOH$, phenyl or benzyl group.

In the formula (VI) above, the alkyl group comprising from 1 to 4 carbon atoms may in particular denote methyl and ethyl groups.

The anionic fixing polymers containing carboxylic or sulfonic groups that are preferred are:

A) copolymers of acrylic or methacrylic acid or salts thereof, including copolymers of acrylic acid and acrylamide, and methacrylic acid/acrylic acid/ethyl acrylate/methyl methacrylate copolymers, more particularly Amerhold DR 25 sold by the company Amerchol, and sodium salts of polyhydroxycarboxylic acids. Mention may also be made of methacrylic acid/ethyl acrylate copolymers, in particular in aqueous dispersion, such as Luviflex Soft and Luvimer MAE, which are sold by the company BASF.

B) copolymers of acrylic or methacrylic acids with a monoethylenic monomer such as ethylene, styrene, vinyl esters and acrylic or methacrylic acid esters, optionally grafted onto a polyalkylene glycol such as polyethylene glycol and optionally crosslinked. Such polymers are described in particular in French patent 1 222 944 and German patent application No. 2 330 956, the copolymers of this type comprising an optionally N-alkylated and/or hydroxyalkylated acrylamide unit in their chain as described especially in Luxembourg patent applications 75370 and 75371. Mention may also be made of copolymers of acrylic acid and $C_1$-$C_4$ alkyl methacrylate.

As another anionic fixing polymer from this class, mention may also be made of the butyl acrylate/acrylic acid/methacrylic acid branched block anionic polymer sold under the name Fixate G-100 L by the company Lubrizol (INCI name AMP-Acrylates/Allyl Methacrylate Copolymer).

C) copolymers derived from crotonic acid, such as those including in their chain vinyl propionate or acetate units, and optionally other monomers such as allyl or methallyl esters, vinyl ethers or vinyl esters of a linear or branched, saturated carboxylic acid with a long hydrocarbon-based chain, such as those containing at least 5 carbon atoms, it being possible optionally for these polymers to be grafted and crosslinked, or else a vinyl, allyl or methallyl ester of an $\alpha$- or $\beta$-cyclic carboxylic acid. Such polymers are described, *inter alia*, in French patents Nos. 1 222 944, 1 580 545, 2 265 782, 2 265 781, 1 564 110 and 2 439 798. Commercial products that fall within this category are the resins 28-29-30, 26-13-14 and 28-13-10 sold by the company National Starch.

Mention may also be made, as copolymer derived from crotonic acid, of crotonic acid/vinyl acetate/vinyl tert-butylbenzoate terpolymers, and more particularly Mexomer PW supplied by the company Chimex.

D) polymers derived from maleic, fumaric or itaconic acids or anhydrides with vinyl esters, vinyl ethers, vinyl halides, phenylvinyl derivatives or acrylic acid and esters thereof; these polymers may be esterified. Polymers of these kinds are described in particular in patents US 2 047 398, 2 723 248 and 2 102 113, and in patent GB 839 805, and in particular are those sold under the names Gantrez® AN or ES by the company ISP.

Polymers also falling within this category are the copolymers of maleic, citraconic and/or itaconic anhydrides and of an allylic or methallylic ester optionally comprising an acrylamide or methacrylamide group, an $\alpha$-olefin, acrylic or methacrylic esters, acrylic or methacrylic acids or vinylpyrrolidone in their chain, the anhydride functions being monoesterified or monoamidated. These polymers are described, for example, in French patents 2 350 384 and 2 357 241 by the Applicant.

E) polyacrylamides comprising carboxylate groups.

F) polymers containing sulfonic groups. These polymers may be polymers comprising vinylsulfonic, styrenesulfonic, naphthalenesulfonic, acrylamidoalkylsulfonic or sulfoisophthalate units.

These polymers may be chosen especially from:

- polyvinylsulfonic acid salts with a molecular mass of between about 1000 and 100 000, and also copolymers with an unsaturated comonomer such as acrylic or methacrylic acids and esters thereof, and also acrylamide or derivatives thereof, vinyl ethers and vinylpyrrolidone;
- polystyrenesulfonic acid salts and sodium salts, having a molecular mass of approximately 500 000 and of about 100 000. These compounds are described in patent FR 2 198 719;

- polyacrylamidesulfonic acid salts such as those mentioned in patent US 4 128 631.

G) grafted anionic silicone polymers.
The grafted silicone polymers used are preferably chosen from polymers containing a non-silicone organic backbone grafted with monomers containing a polysiloxane, polymers containing a polysiloxane backbone grafted with non-silicone organic monomers, and mixtures thereof.
H) anionic polyurethanes, possibly comprising silicone grafts and silicones containing hydrocarbon-based grafts.

By way of examples of fixing polyurethanes, mention may be made, in particular, of the dimethylolpropionic acid/isophorone diisocyanate/neopentyl glycol/polyesterdiol copolymer (also known under the name polyurethane-1, INCI nomenclature) sold under the brand name Luviset® PUR by the company BASF, and the dimethylolpropionic acid/isophorone diisocyanate/neopentyl glycol/polyesterdiol/silicone diamine copolymer (also known under the name polyurethane-6, INCI nomenclature) sold under the brand name Luviset® Si PUR A by the company BASF.
Another anionic polyurethane that may also be used is Avalure UR 450.
Polymers containing sulfoisophthalate groups, such as the polymers AQ55 and AQ48 sold by the company Eastman, may also be used.
According to the invention, the anionic fixing polymers are preferably chosen from acrylic acid copolymers, such as the acrylic acid/ethyl acrylate/N-tert-butylacrylamide terpolymer sold under the name Ultrahold Strong® by the company BASF, methacrylic acid/ethyl acrylate copolymers, in particular in aqueous dispersion, such as Luviflex Soft and Luvimer MAE sold by the company BASF, crotonic acid-derived copolymers, such as the vinyl acetate/vinyl tert-butylbenzoate/crotonic acid terpolymers and the crotonic acid/vinyl acetate/vinyl neododecanoate terpolymers sold under the name Resin 28-29-30 by the company National Starch, polymers derived from maleic, fumaric or itaconic acids or anhydrides with vinyl esters, vinyl ethers, vinyl halides, phenylvinyl derivatives, acrylic acid and its esters, such as the monoesterified methyl vinyl ether/maleic anhydride copolymer sold under the name Gantrez® ES 425 by the company ISP, Luviset Si PUR, Mexomer PW, elastomeric or non-elastomeric anionic polyurethanes, polymers comprising sulfoisophthalate groups, and anionic fixing polymers of the B) class; and even more particularly use is preferably made of the butyl acrylate/acrylic acid/methacrylic acid branched block anionic polymer sold under the name Fixate G-100 L by the company Lubrizol (INCI name AMP-Acrylates/Allyl Methacrylate Copolymer).
The cationic fixing polymers that may be used according to the present invention are preferably chosen from polymers comprising primary, secondary, tertiary and/or quaternary amine groups forming part of the polymer chain or directly attached thereto, and having a molecular weight of between 500 and approximately 5 000 000 and preferably between 1000 and 3 000 000.
Among these polymers, mention may be made more particularly of the following cationic polymers:

(1) Homopolymers or copolymers derived from acrylic or methacrylic esters or amides and comprising at least one of the units of following formulae:

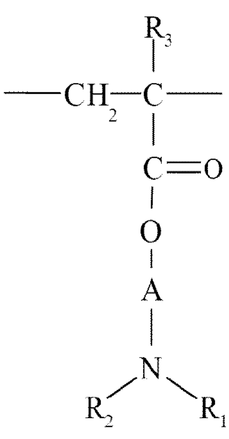 (A) ,

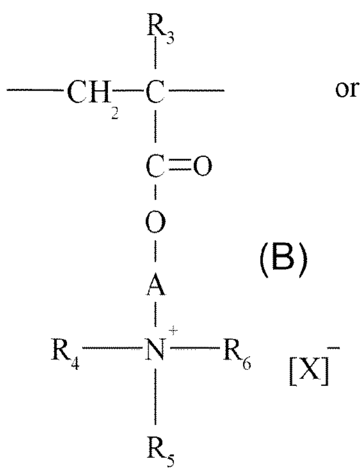 (B) or

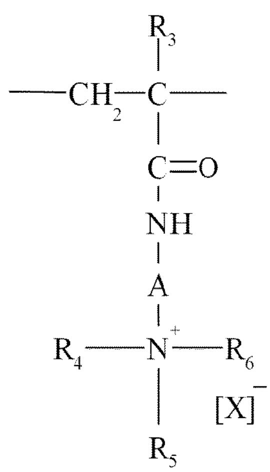 (C)

in which:

$R_3$ denotes a hydrogen atom or a $CH_3$ group;
A is a linear or branched alkyl group comprising from 1 to 6 carbon atoms or a hydroxyalkyl group comprising from 1 to 4 carbon atoms;

$R_4$, $R_5$ and $R_6$, which may be identical or different, represent an alkyl group containing from 1 to 18 carbon atoms, or a benzyl group;
$R_1$ and $R_2$, which may be identical or different, each represent a hydrogen atom or an alkyl group containing from 1 to 6 carbon atoms;
X denotes a methosulfate anion or a halide such as chloride or bromide.

The copolymers of class (1) further contain one or more units deriving from comonomers which may be chosen from the class of acrylamides, methacrylamides, diacetone acrylamides, acrylamides and methacrylamides substituted on the nitrogen by $C_1$-$C_4$ alkyl groups, groups derived from acrylic or methacrylic acids or esters thereof, vinyllactams such as vinylpyrrolidone or vinylcaprolactam, and vinyl esters.
Thus, mention may be made, among these copolymers of class (1), of:

- copolymers of acrylamide and dimethylamino ethyl methacrylate quaternized with dimethyl sulfate or with a dimethyl halide, such as that sold under the name Hercofloc® by the company Hercules,
- copolymers of acrylamide and of methacryloyloxyethyltrimethylammonium chloride, described, for example, in patent application EP-A-080 976 and sold under the name Bina Quat P 100 by the company Ciba Geigy,
- the copolymer of acrylamide and of methacryloyloxyethyltrimethylammonium methosulfate, such as that sold under the name Reten by the company Hercules,
- quaternized or non-quaternized vinylpyrrolidone/dialkylaminoalkyl acrylate or methacrylate copolymers, such as the products sold under the name Gafquat® by the company ISP, for instance Gafquat® 734 or Gafquat® 755, or alternatively the products known as Copolymer® 845, 958 and 937. These polymers are described in detail in French patents 2 077 143 and 2 393 573,
- polymers comprising a fatty chain and comprising a vinylpyrrolidone unit, such as the products sold under the names Styleze W20 and Styleze W10 by the company ISP,
- dimethylaminoethyl methacrylate/vinylcaprolactam/vinylpyrrolidone terpolymers, such as the product sold under the name Gaffix VC 713 by the company ISP, and
- quaternized vinylpyrrolidone/dimethylaminopropylmethacrylamide copolymers, such as the products sold under the name Gafquat® HS 100 by the company ISP.

(2) Cationic guar gums, preferably containing quaternary ammonium, such as those described in US patents 3 589 578 and 4 031 307, such as guar gums containing trialkylammonium cationic groups. Such products are sold in particular under the trade names Jaguar C13 S, Jaguar C 15 and Jaguar C 17 by the company Meyhall.
(3) Quaternary copolymers of vinylpyrrolidone and of vinylimidazole.
(4) Chitosans or salts thereof; the salts that may be used are in particular the acetate, lactate, glutamate, gluconate or pyrrolidonecarboxylate of chitosan.
Among these compounds, mention may be made of chitosan having a degree of deacetylation of 90.5% by weight, sold under the name Kytan Brut Standard by the company Aber Technologies, and chitosan pyrrolidonecarboxylate sold under the name Kytamer® PC by the company Amerchol.
(5) Cationic cellulose derivatives, such as copolymers of cellulose or of cellulose derivatives grafted with a water-soluble monomer comprising a quaternary ammonium and described in particular in the patent US 4 131 576, such as hydroxyalkylcelluloses, for example hydroxymethyl-, hydroxyethyl- or hydroxypropylcelluloses, grafted in particular with a methacryloyloxyethyltrimethylammonium, methacrylamidopropyltrimethylammonium or dimethyldiallylammonium salt.

The commercial products corresponding to this definition are more particularly the products sold under the name Celquat L 200 and Celquat H 100 by the company National Starch, or else Celquat LOR by the company Akzo Nobel, and correspond to polyquaternium-4.

The amphoteric fixing polymers that can be used in accordance with the invention may be chosen from polymers comprising units B and C distributed randomly in the polymer chain, in which B denotes a unit deriving from a monomer comprising at least one basic nitrogen atom and C denotes a unit deriving from an acid monomer comprising one or more carboxylic or sulfonic groups, or alternatively B and C can denote groups deriving from carboxybetaine or sulfobetaine zwitterionic monomers; B and C can also denote a cationic polymer chain comprising primary, secondary, tertiary or quaternary amine groups, in which at least one of the amine groups bears a carboxylic or sulfonic group connected via a hydrocarbon-based group, or alternatively B and C form part of a chain of a polymer containing an ethylenedicarboxylic unit in which one of the carboxylic groups has been made to react with a polyamine comprising one or more primary or secondary amine groups.

The amphoteric fixing polymers corresponding to the definition given above that are more particularly preferred

are chosen from the following polymers:

1) Polymers resulting from the copolymerization of a monomer derived from a vinyl compound bearing a carboxylic group, such as, more particularly, acrylic acid, methacrylic acid, maleic acid, α-chloroacrylic acid, and a basic monomer derived from a substituted vinyl compound containing at least one basic atom, such as, more particularly, dialkylaminoalkyl methacrylate and acrylate, dialkylaminoalkyl methacrylamide and acrylamide. Such compounds are described in United States patent No 3 836 537.
The vinyl compound may also be a dialkyldiallylammonium salt such as diethyldiallylammonium chloride.

2) Polymers containing units which derive:

a) from at least one monomer chosen from acrylamides or methacrylamides which are substituted on the nitrogen by an alkyl group,

b) at least one acidic comonomer containing one or more reactive carboxylic groups, and

c) at least one basic comonomer such as acrylic and methacrylic acid esters containing primary, secondary, tertiary and quaternary amine substituents, and the product of quaternization of dimethylaminoethyl methacrylate with dimethyl or diethyl sulfate.

The N-substituted acrylamides or methacrylamides that are more particularly preferred according to the invention are groups in which the alkyl groups contain from 2 to 12 carbon atoms and more particularly N-ethylacrylamide, N-tert-butylacrylamide, N-tert-octylacrylamide, N-octylacrylamide, N-decylacrylamide, N-dodecylacrylamide and the corresponding methacrylamides.
The acidic comonomers are more particularly chosen from acrylic acid, methacrylic acid, crotonic acid, itaconic acid, maleic acid and fumaric acid and alkyl monoesters, containing 1 to 4 carbon atoms, of maleic or fumaric acids or anhydrides. The preferred basic comonomers are aminoethyl, butylaminoethyl, N,N'-dimethylaminoethyl and N-tert-butylaminoethyl methacrylates. The copolymers of which the CTFA (4th edition, 1991) name is octylacrylamide/acrylates/butylaminoethyl methacrylate copolymer, such as the products sold under the name Amphomer® or Lovocryl® 47 by the company National Starch, are particularly used.

(3) Crosslinked and partially or totally alkylated polyamino amides derived from polyamino amides of general formula:

$$-\!\!\left[-CO-R_4-CO-z-\right]\!\!- \qquad \text{(VII)}$$

in which $R_4$ represents a divalent group derived from a saturated dicarboxylic acid, from a mono- or dicarboxylic aliphatic acid with an ethylenic double bond, from an ester of an alcohol containing 1 to 6 carbon atoms with these acids, or from a group deriving from the addition of any one of said acids with a bis-primary amine or bis-secondary-derived amine, and Z denotes a group of a bis-primary or mono- or bis-secondary polyalkylene-polyamine, and preferably represents:

a) in proportions of from 60 mol% to 100 mol%, the group

$$-NH-\left[-(CH_2)_x-NH-\right]_p- \qquad \text{(VIII)}$$

where x = 2 and p = 2 or 3, or else x = 3 and p = 2,

this group being derived from diethylenetriamine, from triethylenetetramine or from dipropylenetriamine;

b) in proportions of from 0 to 40 mol%, the group (VIII) above, in which x = 2 and p = 1, which derives from ethylenediamine, or the group deriving from piperazine

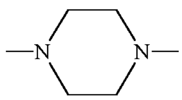

c) in proportions of from 0 to 20 mol%, the group -NH-$(CH_2)_6$-NH- deriving from hexamethylenediamine, these polyaminoamines being crosslinked by addition of a difunctional crosslinking agent chosen from epihalohydrins, diepoxides, dianhydrides, bis-unsaturated derivatives, by means of 0.025 to 0.35 mol of crosslinking agent per amine group of the polyaminoamide, and being alkylated by the action of acrylic acid, chloroacetic acid or an alkane sultone or salts thereof.

The saturated carboxylic acids are preferably chosen from acids having 6 to 10 carbon atoms, such as adipic acid, 2,2,4-trimethyladipic acid and 2,4,4-trimethyladipic acid, terephthalic acid, acids containing an ethylenic double bond, for instance acrylic acid, methacrylic acid and itaconic acid. The alkane sultones used in the alkylation are preferably propane sultone or butane sultone, the salts of the alkylating agents are preferably the sodium or potassium salts.

4) polymers containing zwitterionic units of formula:

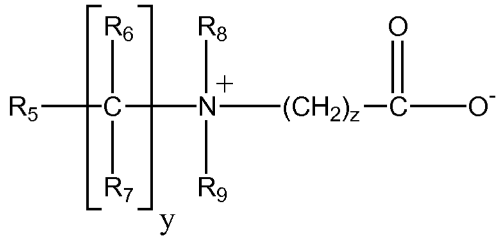

(IX)

in which $R_5$ denotes a polymerizable unsaturated group, such as an acrylate, methacrylate, acrylamide or methacrylamide group, y and z each represent an integer from 1 to 3, $R_6$ and $R_7$ represent a hydrogen atom or a methyl, ethyl or propyl group, $R_8$ and $R_9$ represent a hydrogen atom or an alkyl group such that the sum of the carbon atoms in $R_{10}$ and $R_{11}$ does not exceed 10.
The polymers comprising such units may also comprise units derived from non-zwitterionic monomers such as dimethyl- or diethylaminoethyl acrylate or methacrylate or alkyl acrylates or methacrylates, acrylamides or methacrylamides or vinyl acetate.

5) Polymers derived from chitosan comprising monomer units corresponding to the following formulae:

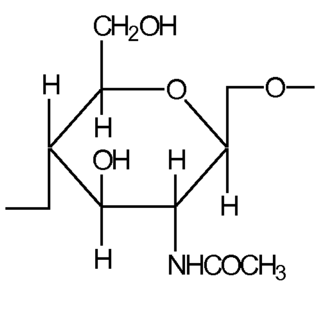

(X)

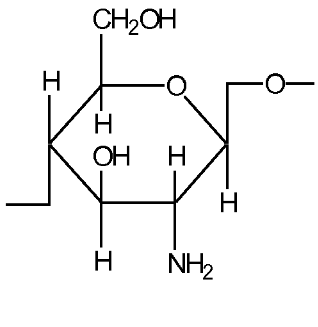

(XI)

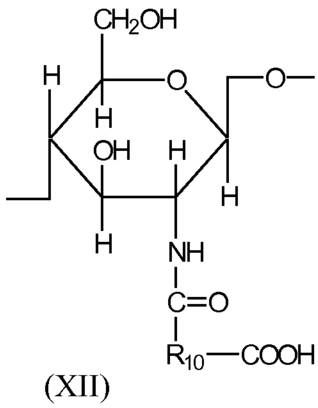

(XII)

the unit (X) being present in proportions of between 0 and 30%, the unit (XI) in proportions of between 5% and 50% and the unit (XII) in proportions of between 30% and 90%, it being understood that, in this unit F, $R_{10}$ represents a group of formula:

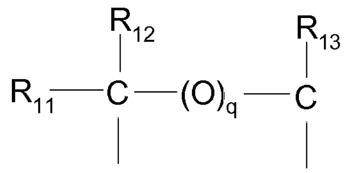

(XIII)

in which, if q = 0, $R_{11}$, $R_{12}$ and $R_{13}$, which may be identical or different, each represent a hydrogen atom, a methyl, hydroxyl, acetoxy or amino residue, a monoalkylamine residue or a dialkylamine residue that are optionally interrupted with one or more nitrogen atoms and/or optionally substituted with one or more amine, hydroxyl, carboxyl, alkylthio or sulfonic groups, an alkylthio residue in which the alkyl group bears an amino residue, at least one of the groups $R_{17}$, $R_{18}$ and $R_{19}$ being, in this case, a hydrogen atom;
or, if q = 1, $R_{11}$, $R_{12}$ and $R_{13}$ each represent a hydrogen atom, and also the salts formed by these compounds with bases or acids.

6) Polymers derived from the N-carboxyalkylation of chitosan.

7) Polymers of units corresponding to the general formula (XIV) described, for example, in French patent 1 400 366:

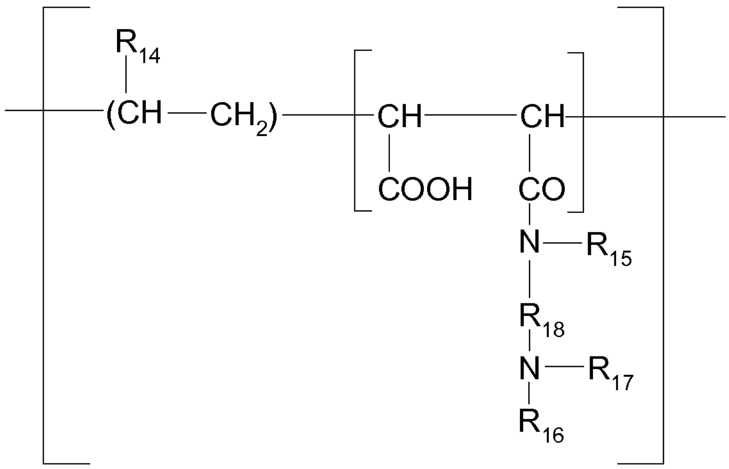

(XIV)

in which $R_{14}$ represents a hydrogen atom or a $CH_3O$, $CH_3CH_2O$, or phenyl group, $R_{15}$ denotes hydrogen or a $C_1$-$C_4$ alkyl group such as methyl and ethyl, $R_{16}$ denotes hydrogen or a $C_1$-$C_4$ alkyl group such as methyl and ethyl, $R_{17}$ denotes a $C_1$-$C_4$ alkyl group such as methyl and ethyl or a group corresponding to the formula: $-R_{18}-N(R_{16})_2$, with $R_{18}$ representing a $-CH_2-CH_2-$, $-CH_2-CH_2-CH_2-$, or $-CH_2-CH(CH_3)-$group and $R_{16}$ having the meanings given above, and also the higher homologues of these groups, containing up to 6 carbon atoms.

8) Amphoteric polymers of type -D-X-D-X-, selected from:

a) polymers obtained by the action of chloroacetic acid or sodium chloroacetate on compounds comprising at least one unit of formula:

-D-X-D-X-D- (XV)

where D denotes a group

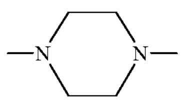

and X denotes the symbol E or E', where E or E', which may be identical or different, denote a divalent group that is an alkylene group with a straight or branched chain containing up to 7 carbon atoms in the main chain, which is unsubstituted or substituted with hydroxyl groups and which can comprise, in addition to oxygen, nitrogen and sulfur atoms, 1 to 3 aromatic and/or heterocyclic rings; the oxygen, nitrogen and sulfur atoms being present in the form of ether, thioether, sulfoxide, sulfone, sulfonium, alkylamine or alkenylamine groups, hydroxyl, benzylamine, amine oxide, quaternary ammonium, amide, imide, alcohol, ester and/or urethane groups;

b) polymers of formula:

-D-X-D-X- (XVI)

where D denotes a group

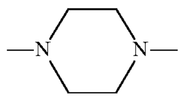

and X denotes the symbol E or E' and at least once E'; E having the meaning given above and E' is a divalent group that is an alkylene group with a straight or branched chain having up to 7 carbon atoms in the main chain, which is unsubstituted or substituted with one or more hydroxyl groups and contains one or more nitrogen atoms, the nitrogen atom being substituted with an alkyl chain that is optionally interrupted with an oxygen atom and necessarily comprising one or more carboxyl functions or one or more hydroxyl functions and betainized by reaction with chloroacetic acid or sodium chloroacetate.

9) ($C_1$-$C_5$)alkyl vinyl ether/maleic anhydride copolymers partially modified by semiamidation with an N,N-dialkylaminoalkylamine such as N,N-dimethylaminopropylamine or by semiesterification with an N,N-dialkanolamine.These copolymers may also comprise other vinyl comonomers such as vinylcaprolactam.

According to a preferred embodiment, the amphoteric fixing polymers that may be used in the invention may be chosen from branched block copolymers comprising:

(a) nonionic units derived from at least one monomer chosen from $C_1$-$C_{20}$ alkyl (meth)acrylates, N-mono-($C_2$-$C_{12}$ alkyl)(meth)acrylamides and N,N-di($C_2$-$C_{12}$ alkyl)(meth)acrylamides,

(b) anionic units derived from at least one monomer chosen from acrylic acid and methacrylic acid, and

(c) polyfunctional units derived from at least one monomer comprising at least two polymerizable unsaturated functional groups,

and preferably having a structure consisting of hydrophobic blocks onto which are fixed, via polyfunctional units (c), several blocks that are more hydrophilic.

Preferably, the amphoteric polymers have at least two glass transition temperatures (Tg), at least one of which is greater than 20°C and the other is less than 20°C.

The preferred amphoteric polymers are polymers comprising units deriving:

a) from at least one monomer chosen from acrylamides or methacrylamides which are substituted on the nitrogen by an alkyl group,

b) at least one acidic comonomer containing one or more reactive carboxylic groups, and

c) at least one basic comonomer such as acrylic and methacrylic acid esters containing primary, secondary, tertiary and quaternary amine substituents, and the product of quaternization of dimethylaminoethyl methacrylate with dimethyl or diethyl sulfate.

Mention may be made in particular of the polymers sold under the name Amphomer by the company National Starch.

The nonionic fixing polymers that may be used according to the present invention are chosen, for example, from:

- polyalkyloxazolines;
- vinyl acetate homopolymers;
- vinyl acetate copolymers, for instance copolymers of vinyl acetate and of acrylic ester, copolymers of vinyl acetate and of ethylene, or copolymers of vinyl acetate and of maleic ester, for example of dibutyl maleate,
- acrylic ester homopolymers and copolymers, for instance copolymers of alkyl acrylates and of alkyl methacrylates, such as the products provided by the company Röhm & Haas under the names Primal® AC-261 K and Eudragit® NE 30 D, by the company BASF under the name 8845, or by the company Hoechst under the name Appretan® N9212,
- copolymers of acrylonitrile and of a nonionic monomer chosen, for example, from butadiene and alkyl (meth)acrylates, such as the products provided under the name CJ 0601 B by the company Röhm & Haas,
- styrene homopolymers,
- styrene copolymers, for instance copolymers of styrene and of alkyl (meth)acrylate, such as the products Mowilith® LDM 6911, Mowilith® DM 611 and Mowilith® LDM 6070 provided by the company Hoechst, the products Rhodopas® SD 215 and Rhodopas® DS 910 provided by the company Rhone-Poulenc, copolymers of styrene, of alkyl methacrylate and of alkyl acrylate, copolymers of styrene and of butadiene, or copolymers of styrene, of butadiene and of vinylpyridine,
- polyamides,
- vinyllactam homopolymers such as vinylpyrrolidone homopolymers and such as the polyvinylcaprolactam sold under the name Luviskol® Plus by the company BASF,
- vinyllactam copolymers, such as a poly(vinylpyrrolidone/vinyllactam) copolymer sold under the trade name Luvitec® VPC 55K65W by the company BASF, poly(vinylpyrrolidone/vinyl acetate) copolymers, such as those sold under the name PVPVA® S630L by the company ISP, Luviskol® VA 73, VA 64, VA 55, VA 37 and VA 28 by the company BASF and poly(vinylpyrrolidone/vinyl acetate/vinyl propionate) terpolymers, for instance the product sold under the name Luviskol® VAP 343 by the company BASF, and
- poly(vinyl alcohols).

The alkyl groups of the nonionic polymers mentioned above preferably contain from 1 to 6 carbon atoms.

Preferably, the fixing polymer(s) are chosen from nonionic fixing polymers and anionic fixing polymers. More preferentially, the fixing polymer(s) are chosen from nonionic fixing polymers such as those described above, and more preferentially from vinyllactam copolymers, such as poly(vinylpyrrolidone/vinyllactam) copolymers and poly(vinylpyrrolidone/vinyl acetate) copolymers.

The fixing polymer(s) other than the vinylformamide/vinylamine copolymers may be present in an amount ranging from 0.1% to 20% by weight, preferably from 0.2% to 15% by weight and even more preferentially from 0.3% to 10% by weight, relative to the total weight of the composition.

Preferably, the ratio of the amounts by weight of vinylformamide/vinylamine copolymer(s):fixing polymer(s) other than the vinylformamide/vinylamine copolymer(s) ranges from 0.01 to 10, more preferentially from 0.05 to 5 and better still from 0.1 to 2.

The composition according to the invention generally comprises water, which advantageously represents at least 5% by weight of its total weight. Preferably, the composition according to the invention comprises from 20% to 98% by weight, preferably from 30% to 97% by weight and better still from 40% to 95% by weight of water, relative to the total weight of the composition.

According to a particularly preferred embodiment, the composition according to the invention also comprises one or more propellants. The composition according to the invention is thus sprayable in foam form.

As propellants that may be used, mention may be made of the gases usually used in the field of cosmetics, in particular: optionally halogenated volatile hydrocarbons, for example n-butane, propane, isobutane and pentane, and halogenated derivatives thereof; carbon dioxide; air; dimethyl ether; nitrogen, and mixtures thereof.

These agents are generally gaseous at room temperature and atmospheric pressure.

Preferably, the propellant(s) are chosen from dimethyl ether and alkanes, in particular n-butane, propane and isobutane, and mixtures thereof.

For the packaging of the composition according to the invention, the propellant(s) are generally under pressure, more particularly at least partially in liquid form.

The propellant(s) may represent from 0.1% to 40% by weight, preferably from 1% to 30% by weight and more particularly from 3% to 20% by weight, relative to the total weight of the composition according to the invention.

The composition according to the present invention may also comprise one or more surfactants other than the particular surfactants described above, especially such as: one or more nonionic surfactants other than oxyalkylenated fatty alcohols; one or more anionic surfactants; one or more amphoteric or zwitterionic surfactants.

According to a preferred embodiment, the composition according to the invention is non-washing, i.e. its total content of anionic surfactants and of nonionic surfactants is less than or equal to 5% by weight, relative to the total weight of the composition.

The composition may also comprise one or more water-soluble organic solvents (solubility of greater than or equal to 5% by weight in water at 25°C and at atmospheric pressure).

According to a preferred embodiment, the composition according to the invention comprises one or more water-soluble organic solvents chosen from linear or branched and preferably saturated monoalcohols and diols, comprising 2 to 10 carbon atoms, such as ethyl alcohol, isopropyl alcohol, hexylene glycol (2-methyl-2,4-pentanediol), neopentyl glycol, 3-methyl-1,5-pentanediol, butylene glycol, dipropylene glycol and propylene glycol; aromatic alcohols such as phenylethyl alcohol; polyols containing more than two hydroxyl functions, such as glycerol; polyol ethers, for instance ethylene glycol monomethyl, monoethyl and monobutyl ether, propylene glycol or ethers thereof, for instance propylene glycol monomethyl ether; and also diethylene glycol alkyl ethers, especially $C_1$ to $C_4$ alkyl ethers, for instance diethylene glycol monoethyl ether or monobutyl ether, alone or as a mixture. They are preferably chosen from saturated diols comprising 2 to 10 carbon atoms and glycerol.

The water-soluble organic solvents, when they are present, generally represent from 0.01% to 20% by weight, preferably from 0.1% to 15% by weight and more preferentially from 0.2% to 10% by weight relative to the total weight of the composition.

The composition according to the invention may also comprise one or more amino silicones.

For the purposes of the present invention, the term "amino silicone" means any silicone comprising at least one primary, secondary or tertiary amine function or a quaternary ammonium group.

Preferably, the amino silicone(s) used in the composition according to the present invention comprise in their structure at least 4 silicon atoms.

Throughout the text hereinbelow, the term "silicone" is intended to denote, in accordance with what is generally accepted, any organosilicon polymers or oligomers of linear or cyclic, branched or crosslinked structure, of variable molecular weight, obtained by polymerization and/or polycondensation of suitably functionalized silanes, and constituted essentially of a repetition of main units in which the silicon atoms are linked together via oxygen atoms (siloxane bond -Si-O-Si-), optionally substituted hydrocarbon-based groups being directly linked via a carbon atom to said silicon atoms. The hydrocarbon-based groups that are the most common are alkyl groups, especially $C_1$-$C_{10}$ alkyl groups and in particular methyl, fluoroalkyl groups the alkyl part of which is $C_1$-$C_{10}$, and aryl groups and in particular phenyl.

The amino silicones used in the composition according to the present invention may be chosen more particularly from:

(a) the compounds corresponding to formula (XVII) below:

$$(R^1)_a(T)_{3-a}\text{-}Si[OSi(T)_2]_n\text{-}[OSi(T)_b(R^1)_{2-b}]_m\text{-}OSi(T)_{3-a}\text{-}(R^1)_a \qquad \text{(XVII)}$$

in which:

T is a hydrogen atom or a phenyl or hydroxyl (-OH), or $C_1$-$C_8$ alkyl, and preferably methyl, or $C_1$-$C_8$ alkoxy, preferably methoxy, group,
a denotes the number 0 or an integer from 1 to 3, and preferably 0,
b denotes 0 or 1, and in particular 1,
m and n are numbers such that the sum (n + m) can range especially from 1 to 2000 and in particular from 50 to 150, n possibly denoting a number from 0 to 1999 and especially from 49 to 149, and m possibly denoting a number from 1 to 2000 and especially from 1 to 10,
$R^1$ is a monovalent group of formula $-C_qH_{2q}L$ in which q is a number from 2 to 8 and L is an optionally quaternized amine group chosen from the following groups:

$-N(R^2)-CH_2-CH_2-N(R^2)_2$,
$-N(R^2)_2$,
$-N^+(R^2)_3Q^-$,
$-N^+(R^2)(H)_2\ Q^-$,
$-N^+(R^2)_2HQ^-$,
$-N(R^2)-CH_2-CH_2-N^+(R^2)(H)_2Q^-$,

in which $R^2$ may denote a hydrogen atom, a phenyl, benzyl or saturated monovalent hydrocarbon-based group, for example a $C_1$-$C_{20}$ alkyl group, and $Q^-$ represents a halide ion, for instance fluoride, chloride, bromide or iodide.

In particular, the amino silicones corresponding to the definition of formula (XVII) are chosen from the compounds corresponding to formula (XVIII) below:

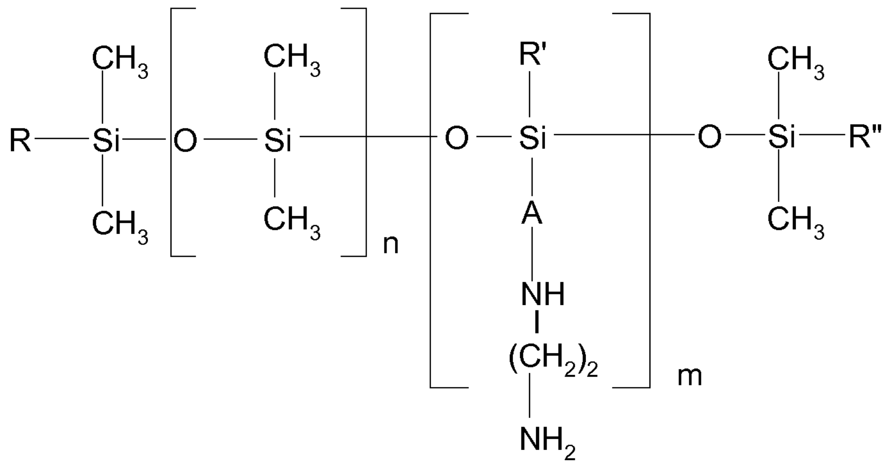

(XVIII)

in which R, R' and R", which may be identical or different, denote a $C_1$-$C_4$ alkyl group, preferably $CH_3$; a $C_1$-$C_4$ alkoxy group, preferably methoxy; or OH; A represents a linear or branched, $C_3$-$C_8$ and preferably $C_3$-$C_6$, alkylene group; m and n are integers dependent on the molecular weight and the sum of which is between 1 and 2000.

According to a first possibility, R, R' and R", which may be identical or different, each represent a $C_1$-$C_4$ alkyl or hydroxyl group, A represents a $C_3$ alkylene group and m and n are such that the weight-average molecular weight of the compound is between approximately 5000 and 500 000. The compounds of this type are named "amodimethicone" in the CTFA dictionary.

According to a second possibility, R, R' and R", which may be identical or different, each represent a $C_1$-$C_4$ alkoxy or hydroxyl group, at least one of the groups R and R" is an alkoxy group and A represents a $C_3$ alkylene group. The hydroxyl/alkoxy mole ratio is preferably between 0.2/1 and 0.4/1 and advantageously equal to 0.3/1. Moreover, m and n are such that the weight-average molecular weight of the compound is between 2000 and $10^6$. More particularly, n is between 0 and 999 and m is between 1 and 1000, the sum of n and m being between 1 and 1000.

In this category of compounds, mention may be made, *inter alia,* of the product Belsil® ADM 652 sold by Wacker.

According to a third possibility, R and R", which are different, each represent a $C_1$-$C_4$ alkoxy or hydroxyl group, at least one of the groups R and R" being an alkoxy group, R' representing a methyl group and A representing a $C_3$ alkylene group. The hydroxyl/alkoxy mole ratio is preferably between 1/0.8 and 1/1.1 and advantageously is equal to 1/0.95. Moreover, m and n are such that the weight-average molecular weight of the compound is between 2000 and 200 000. More particularly, n is between 0 and 999 and m is between 1 and 1000, the sum of n and m being between 1 and 1000.

More particularly, mention may be made of the product Fluid WR® 1300 sold by the company Wacker.

It should be noted that the molecular weight of these silicones is determined by gel permeation chromatography (room temperature; polystyrene standard; μ styragem columns; THF eluent; flow rate of 1 mm/m; 200 μl of a solution containing 0.5% by weight of silicone in THF are injected, and detection is performed by refractometry and UV-metry).

A product corresponding to the definition of formula (XVII) is in particular the polymer known in the CTFA dictionary as "trimethylsilyl amodimethicone", corresponding to formula (XIX) below:

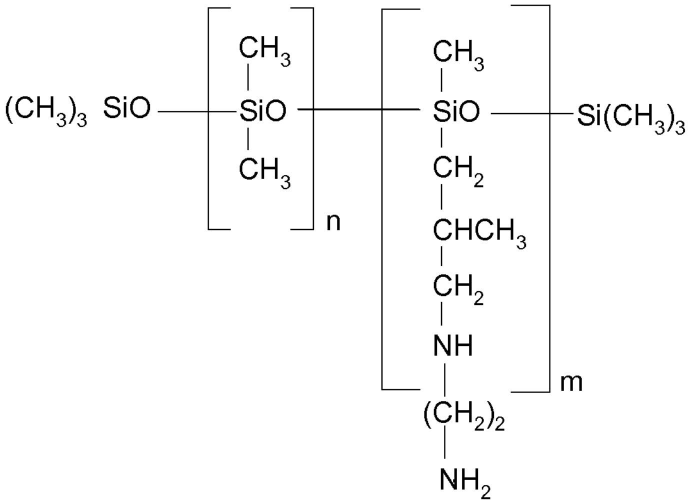

(XIX)

in which n and m have the meanings given above in accordance with formula (XVII).

Such compounds are described, for example, in patent EP 95238. A compound of formula (XVII) is sold, for example, under the name Q2-8220 by the company OSI.

(b) the compounds corresponding to formula (XX) below:

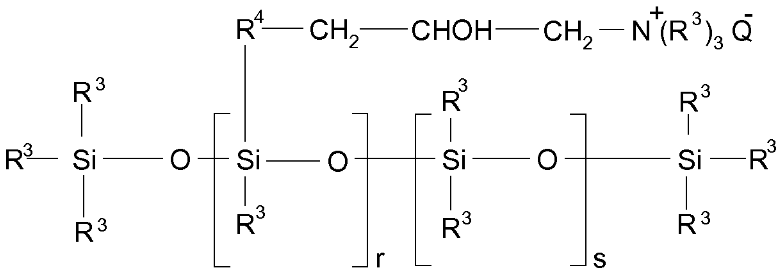

(XX)

in which:

- $R_3$ represents a monovalent $C_1$-$C_{18}$ hydrocarbon-based group, and in particular a $C_1$-$C_{18}$ alkyl or $C_2$-$C_{18}$ alkenyl group, for example methyl,
- $R^4$ represents a divalent hydrocarbon-based group, in particular a $C_1$-$C_{18}$ alkylene group or a divalent $C_1$-$C_{18}$, for example $C_1$-$C_8$, alkyleneoxy group,
- $Q^-$ is a halide ion, in particular chloride,
- r represents a mean statistical value from 2 to 20 and in particular from 2 to 8,
- s represents a mean statistical value from 20 to 200 and in particular from 20 to 50.

Such compounds are described more particularly in patent US 4 185 087.

A compound falling within this class is the product sold by Union Carbide under the name Ucar Silicone ALE 56.

c) quaternary ammonium silicones, especially of formula (XXI):

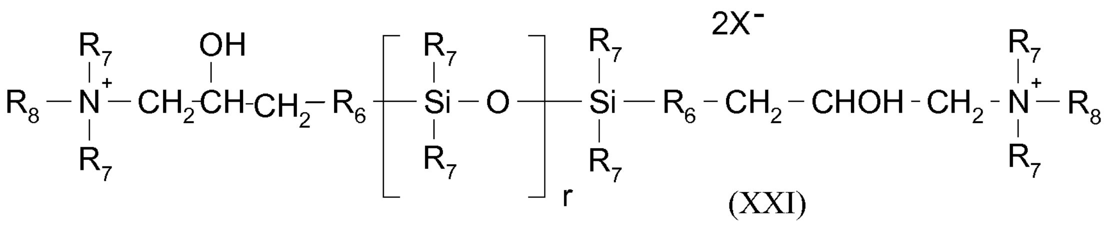

(XXI)

in which:

$R_7$, which may be identical or different, represent a monovalent hydrocarbon-based group having from 1 to 18 carbon atoms, and in particular a $C_1$-$C_{18}$ alkyl group, a $C_2$-$C_{18}$ alkenyl group or a ring comprising 5 or 6 carbon atoms; for example a methyl group,
$R_6$ represents a divalent hydrocarbon-based group, in particular a $C_1$-$C_{18}$ alkylene group or a divalent $C_1$-$C_{18}$, for example $C_1$-$C_8$, alkyleneoxy group linked to the Si via an Si-C bond,
$R_8$, which may be identical or different, each represent a hydrogen atom, a monovalent hydrocarbon-based group containing from 1 to 18 carbon atoms, and in particular a $C_1$-$C_{18}$ alkyl group, a $C_2$-$C_{18}$ alkenyl group or an -$R_6$-NHCO$R_7$ group;
$X^-$ is an anion, such as a halide ion, in particular chloride, or an organic acid salt (acetate, etc.),
r represents a mean statistical value from 2 to 200 and in particular from 5 to 100.

These silicones are described, for example, in patent application EP-A 0 530 974.
(d) the amino silicones of formula (XXII):

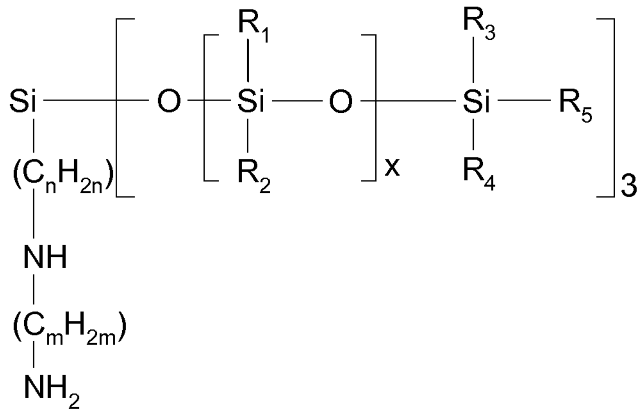

(XXII)

in which:

- $R_1$, $R_2$, $R_3$ and $R_4$, which may be identical or different, each denote a $C_1$-$C_4$ alkyl group or a phenyl group,
- $R_5$ denotes a $C_1$-$C_4$ alkyl group or a hydroxyl group,
- n is an integer ranging from 1 to 5,
- m is an integer ranging from 1 to 5, and
- x is chosen such that the amine number is between 0.01 and 1 meq/g.

When these compounds are used, one particularly advantageous embodiment involves their combined use with cationic and/or nonionic surfactants.
By way of example, use may be made of the product sold under the name Cationic Emulsion DC939 by Dow Corning, which comprises, besides amodimethicone, a cationic surfactant, namely trimethylcetylammonium chloride, and a nonionic surfactant of formula $C_{13}H_{27}$-$(OC_2H_4)_{12}$-OH, known under the CTFA name Trideceth-12.
Another commercial product that may be used according to the invention is the product sold under the name Dow Corning Q2 7224 by Dow Corning, comprising, in combination, the trimethylsilyl amodimethicone of formula (XIX) described above, a nonionic surfactant of formula: $C_8H_{17}$-$C_6H_4$-$(OCH_2CH_2)_{40}$-OH, known under the CTFA name Octoxynol-40, a second nonionic surfactant of formula: $C_{12}H_{25}$-$(OCH_2$-$CH_2)_6$-OH, known under the CTFA name Isolaureth-6, and propylene glycol.
Another commercial product that may be used according to the invention is the product sold under the name Wacker-Belsil ADM LOG 1, sold by the company Wacker, comprising, in microemulsion form, an amodimethicone of formula (XVIII) in combination with Trideceth-5 and Trideceth-10.
It is also possible to use the product sold under the trade name Xiameter MEM 8299 emulsion by Dow Corning.
Other amino silicones may be used within the context of the invention, such as the product which is referenced in the CTFA dictionary under the name Polysilicone-9.
Preferably, the amino silicone(s) used in the composition according to the invention are chosen from the amino silicones corresponding to formula (XVII), and even more particularly from the amino silicones of formula (XVIII) or (XIX),

better still from the amino silicones of formula (XVIII).

Preferably, the amino silicone(s) present in the composition according to the invention are non-phenyl silicones.

The content of amino silicones in the composition according to the invention may range from 0.01% to 10% by weight, preferably from 0.1% to 5% by weight and even more preferentially from 0.15% to 3% by weight relative to the total weight of the composition.

The composition according to the invention may also comprise one or more additives other than the compounds of the invention.

As additives that may be used in accordance with the invention, mention may be made of anionic, nonionic or amphoteric polymers or mixtures thereof, other than the fixing polymers described previously, agents for preventing hair loss, fatty substances, vitamins and provitamins including panthenol, sunscreens, mineral or organic pigments, sequestrants, plasticizers, solubilizers, acidifying agents, mineral or organic thickeners, especially polymeric thickeners, opacifiers or nacreous agents, antioxidants, hydroxy acids, fragrances and preserving agents.

Needless to say, those skilled in the art will take care to select this or these optional additional compound(s) such that the advantageous properties intrinsically associated with the composition according to the invention are not, or are not substantially, adversely affected by the envisaged addition(s).

The above adjuvants may generally be present in an amount, for each of them, of between 0 and 20% by weight relative to the total weight of the composition.

According to a preferred embodiment, the composition according to the invention is packaged under pressure, in an aerosol device, for example a monobloc device, which comprises a dispensing means and a container.

The dispensing means is generally formed from a dispensing valve controlled by a dispensing head, which itself comprises a nozzle via which the composition of the invention is sprayed in mousse form.

The container containing the pressurized composition may be opaque or transparent. It may be made of glass, polymer or metal, and may optionally be coated with a protective varnish coat.

A subject of the invention is also the use of the composition as defined previously for shaping the hair.

Finally, a subject of the invention is a process for styling keratin fibres, preferably the hair, comprising the application to said fibres of the composition as defined previously. This application may optionally be followed by rinsing after an optional leave-on time.

Preferably, the application is performed in non-rinsed mode.

In a first embodiment of the process according to the invention, the composition is applied to wet hair.

In a second embodiment of the process according to the invention, the composition is applied to dry hair.

The following examples are given purely by way of illustration of the present invention.

EXAMPLES:

The following compositions were prepared from the ingredients indicated in the tables below, in which all the amounts are indicated as weight percentages of active material (AM) relative to the total weight of the composition.

Composition A:

| Ingredients | Content (weight % of AM) |
|---|---|
| Vinylpyrrolidone/vinyl acetate copolymer (60/40) as an aqueous 50% solution | 3.2 |
| Polydimethylsiloxane containing aminoethyl aminopropyl groups, containing methoxy and/or hydroxyl and alpha-omega silanol functions, as a cationic 60% aqueous emulsion | 0.5 |
| Vinylamine/N-vinylformamide copolymer (degree of hydrolysis 30%, MW 340 000) as an aqueous 16% solution | 0.7 |
| Oxyethylenated lauryl alcohol (4 EO) | 0.2 |
| Fragrance, preserving agent | qs |
| Cetyltrimethylammonium chloride as an aqueous 25% solution | 0.2 |
| Propylene glycol | 1.8 |
| Oxyethylenated (40 EO) hydrogenated castor oil | 0.5 |
| Isobutane/propane/butane mixture | 6 |
| Purified dimethyl ether | 2 |

(continued)

| Ingredients | Content (weight % of AM) |
|---|---|
| Deionized water | qs 100 |

Composition B:

| Ingredients | Content (weight % of AM) |
|---|---|
| Vinylpyrrolidone/vinyl acetate copolymer (60/40) as an aqueous 50% solution | 3.2 |
| Polydimethylsiloxane containing aminoethyl aminopropyl groups, containing methoxy and/or hydroxyl and alpha-omega silanol functions, as a cationic 60% aqueous emulsion | 0.5 |
| Vinylamine/N-vinylformamide copolymer (degree of hydrolysis 30%, MW 340 000) as an aqueous 16% solution | 0.7 |
| Oxyethylenated lauryl alcohol (4 EO) | 0.2 |
| Cetyltrimethylammonium chloride as an aqueous 25% solution | 0.2 |
| Propylene glycol | 1.8 |
| Oxyethylenated (40 EO) hydrogenated castor oil | 0.5 |
| Isobutane/propane/butane mixture | 8 |
| Fragrance, preserving agent | qs |
| Deionized water | qs 100 |

Composition C:

| Ingredients | Content (weight % of AM) |
|---|---|
| Vinylpyrrolidone/vinyl acetate copolymer (60/40) as an aqueous 50% solution | 3.2 |
| Vinylamine/N-vinylformamide copolymer (degree of hydrolysis 30%, MW 340 000) as an aqueous 16% solution | 0.7 |
| Oxyethylenated lauryl alcohol (4 EO) | 0.2 |
| Cetyltrimethylammonium chloride as an aqueous 25% solution | 0.2 |
| Propylene glycol | 1.8 |
| Oxyethylenated (40 EO) hydrogenated castor oil | 0.5 |
| Glycerol | 0.9 |
| Fragrance, preserving agent | qs |
| Purified dimethyl ether | 2 |
| Isobutane/propane/butane mixture | 6 |
| Deionized water | qs 100 |

Composition D:

| Ingredients | Content (weight % of AM) |
|---|---|
| Vinylpyrrolidone/vinyl acetate copolymer (60/40) as an aqueous 50% solution | 3.2 |

(continued)

| Ingredients | Content (weight % of AM) |
|---|---|
| Oxyethylenated lauryl alcohol (4 EO) | 0.2 |
| Vinylamine/N-vinylformamide copolymer (degree of hydrolysis 30%, MW 340 000) as an aqueous 16% solution | 0.7 |
| Propylene glycol | 1.8 |
| Cetyltrimethylammonium chloride as an aqueous 25% solution | 0.2 |
| Oxyethylenated (40 EO) hydrogenated castor oil | 0.5 |
| Fragrance, preserving agent | qs |
| Glycerol | 0.9 |
| Isobutane/propane/butane mixture | 8 |
| Deionized water | qs 100 |

The above compositions were packaged in aerosol devices.

On spraying, firm, creamy mousses are obtained, which spread easily on the head of hair and give good styling and cosmetic properties, in particular with long-lasting fixing and a pleasant feel. The hair is soft, and has volume. The hairstyle obtained is natural, with no helmet effect.

Comparative examples:

The following compositions were prepared, using the ingredients detailed in the tables below, in which all the amounts are indicated as weight percentages of active material (AM) relative to the total weight of the composition.

| Ingredients | Composition E (invention) | Compistion F (comparative) |
|---|---|---|
| Vinylpyrrolidone/vinyl acetate copolymer (60/40) as an aqueous 50% solution | 3.2 | 3.2 |
| Vinylamine/N-vinylformamide copolymer (degree of hydrolysis 30%, MW 340 000) as an aqueous 16% solution | 0.7 | 0.7 |
| Oxyethylenated lauryl alcohol (4 EO) | 0.2 | - |
| Oxyethylenated (40 EO) hydrogenated castor oil | 0.5 | 0.7 |
| Fragrance, preserving agent | qs | qs |
| Cetyltrimethylammonium chloride as an aqueous 25% solution | 0.2 | 0.2 |
| Propylene glycol | 1.8 | 1.8 |
| Isobutane/propane/butane mixture | 8 | 8 |
| Deionized water | qs 100 | qs 100 |

The above compositions were packaged in aerosol devices.

The aerosol device is equipped with a Precision P14105 valve and a Precision DMPR229 diffuser.

The firmness of the mousse obtained on spraying the two compositions above was determined in the following manner:

A standardized cone is introduced into the mousse and an amount of mousse is then sucked into the cone at a rate of 50 microlitres/second for 20 seconds.

The firmness index (i) is represented by the equation:

$$(i) = P_0 - P_{max\ suct.}$$

where Po = initial pressure on introduction of the cone (in Pa)
$P_{max\ suct.}$ = maximum pressure peak measured during suction into the cone (pressure reduction) (in Pa)

The greater the value of (i), the greater the firmness of the mousse.
For each of the compositions (E) and (F), eight measurements were taken simultaneously on a foam sample.
The mean firmness indices obtained are given below:

| | Firmness index of the foam (i) |
|---|---|
| Composition (E) according to the invention | 911.96 ± 45.39 |
| Comparative composition (F) | 725.93 ± 69.52 |

The foam corresponding to the composition (E) has a significantly higher mean firmness index than that of the foam of the composition (F): the foam obtained with composition (E) is thus firmer than that obtained with composition (F).
A firm foam reflects improved working qualities as regards the uptake of the foam in the hands and thus the distribution over the hair.

## Claims

1. Composition comprising:

    - one or more vinylformamide/vinylamine copolymers comprising:

        - vinylamine units of formula (IA):

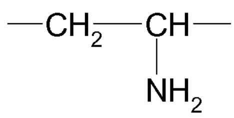
(IA),

        and
        - vinylformamide units of formula (IB):

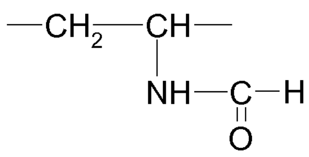
(IB);

    - one or more fixing polymers other than vinylformamide/vinylamine copolymers;
    - one or more nonionic surfactants chosen from oxyalkylenated fatty alcohols comprising a linear or branched, saturated or unsaturated alkyl chain containing at least 8 carbon atoms; and
    - one or more cationic surfactants.

2. Composition according to the preceding claim, **characterized in that** the vinylformamide/vinylamine copolymer(s) comprise from 10 mol% to 95 mol% of units of formula (IA) and from 90 mol% to 5 mol% of units of formula (IB).

3. Composition according to either of the preceding claims, **characterized in that** the vinylformamide/vinylamine copolymer(s) comprise from 10 mol% to 60 mol% of units of formula (IA) and more particularly from 20 mol% to 40 mol%.

4. Composition according to any one of the preceding claims, **characterized in that** the vinylformamide/vinylamine copolymer(s) comprise from 30 mol% to 90 mol% of units of formula (IB) and more particularly from 60 mol% to 80 mol%.

**5.** Composition according to any one of the preceding claims, **characterized in that** the vinylformamide/vinylamine copolymer(s) are formed solely from units of formula (IA) and units of formula (IB).

**6.** Composition according to any one of the preceding claims, **characterized in that** the vinylformamide/vinylamine copolymer(s) are present in proportions ranging from 0.01% to 20% by weight, more preferentially from 0.1% to 10% by weight and more particularly from 0.15% to 5% by weight, relative to the total weight of the composition.

**7.** Composition according to any one of the preceding claims, **characterized in that** the fixing polymers other than vinylformamide/vinylamine copolymers are chosen from nonionic fixing polymers and anionic fixing polymers, preferably from nonionic fixing polymers, even more preferentially from vinyllactam copolymers, such as poly(vinylpyrrolidone/vinyllactam) copolymers and poly(vinylpyrrolidone/vinyl acetate) copolymers.

**8.** Composition according to any one of the preceding claims, **characterized in that** the fixing polymers other than vinylformamide/vinylamine copolymers are present in an amount ranging from 0.1% to 20% by weight, preferably from 0.2% to 15% by weight and even more preferentially from 0.3% to 10% by weight, relative to the total weight of the composition.

**9.** Composition according to any one of the preceding claims, **characterized in that** the oxyalkylenated fatty alcohol(s) comprise fatty alcohols containing 10 to 24 carbon atoms, preferably from 12 to 22 carbon atoms, and from 2 to 20 oxyalkylene units, better still from 3 to 10 oxyalkylene units, even better still from 3 to 6 oxyalkylene units, preferably oxyethylene units.

**10.** Composition according to the preceding claim, **characterized in that** the oxyalkylenated fatty alcohol is lauryl alcohol containing 4 oxyethylene units.

**11.** Composition according to any one of the preceding claims, **characterized in that** its content of nonionic surfactants chosen from oxyalkylenated, preferably oxyethylenated, fatty alcohols ranges from 0.01% to 5% by weight, preferably from 0.05% to 2% by weight and more preferentially from 0.1% to 1% by weight, relative to the total weight of the composition.

**12.** Composition according to any one of the preceding claims, **characterized in that** the cationic surfactant(s) are chosen from:

- those corresponding to the general formula (II) below:

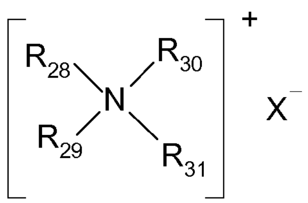

(II)

in which the groups $R_{28}$ to $R_{31}$, which may be identical or different, represent a linear or branched aliphatic group, comprising from 1 to 30 carbon atoms, or an aromatic group such as aryl or alkylaryl, at least one of the groups $R_{28}$ to $R_{31}$ denoting a group comprising from 8 to 30 carbon atoms, preferably from 12 to 24 carbon atoms;

- quaternary ammonium salts of imidazole, for instance those of formula (III) below:

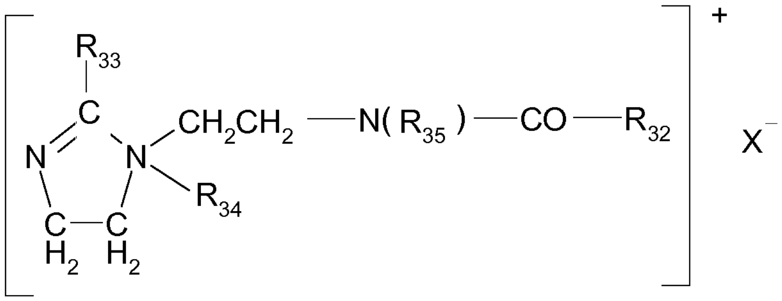

(III)

in which:

- $R_{32}$ represents an alkenyl or alkyl group comprising from 8 to 30 carbon atoms,
- $R_{33}$ represents a hydrogen atom, a $C_1$ to $C_4$ alkyl group or an alkenyl or alkyl group comprising from 8 to 30 carbon atoms,
- $R_{34}$ represents a $C_1$ to $C_4$ alkyl group,
- $R_{35}$ represents a hydrogen atom or a $C_1$ to $C_4$ alkyl group,
- $X^-$ is an anion chosen from the group of halides, phosphates, acetates, lactates, alkyl sulfates, alkyl- or alkylaryl-sulfonates in which the alkyl and aryl groups preferably comprise, respectively, from 1 to 20 carbon atoms and from 6 to 30 carbon atoms;

- the quaternary diammonium or triammonium salts of formula (IV):

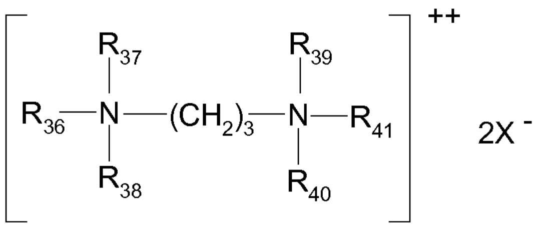

(IV)

in which:

- $R_{36}$ denotes an alkyl radical comprising from about 16 to 30 carbon atoms, which is optionally hydroxylated and/or interrupted with one or more oxygen atoms,
- $R_{37}$ is chosen from hydrogen and an alkyl radical comprising from 1 to 4 carbon atoms or a group $(R_{36a})(R_{37a})(R_{38a})N-(CH_2)_3$,
- $R_{36a}$, $R_{37a}$, $R_{38a}$, $R_{38}$, $R_{39}$, $R_{40}$ and $R_{41}$, which may be identical or different, are chosen from hydrogen and an alkyl radical comprising from 1 to 4 carbon atoms, and
- $X^-$ is an anion chosen from the group of halides, acetates, phosphates, nitrates and methyl sulfates;

- quaternary ammonium salts containing at least one ester function, such as those of formula (V) below:

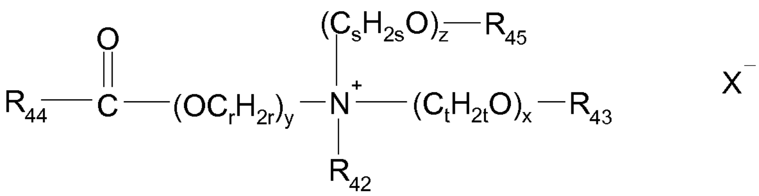

(V)

in which:

- $R_{42}$ is chosen from $C_1$ to $C_6$ alkyl groups and $C_1$ to $C_6$ hydroxyalkyl or dihydroxyalkyl groups;

- $R_{43}$ is chosen from:
- the group

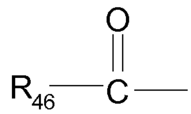

- groups $R_{47}$ which are linear or branched, saturated or unsaturated $C_1$ to $C_{22}$ hydrocarbon-based groups,
- a hydrogen atom,
- $R_{45}$ is chosen from:
- the group

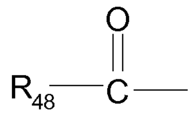

- groups $R_{49}$ which are linear or branched, saturated or unsaturated $C_1$ to $C_6$ hydrocarbon-based groups,
- a hydrogen atom,
- $R_{44}$, $R_{46}$ and $R_{48}$, which may be identical or different, are chosen from linear or branched, saturated or unsaturated $C_7$ to $C_{21}$ hydrocarbon-based groups;
- r, s and t, which may be identical or different, are integers ranging from 2 to 6;
- y is an integer ranging from 1 to 10;
- x and z, which may be identical or different, are integers ranging from 0 to 10;
- $X^-$ is a simple or complex, organic or inorganic anion;

with the proviso that the sum x + y + z is from 1 to 15, that when x is 0, then $R_{43}$ denotes $R_{47}$ and that when z is 0, then $R_{45}$ denotes $R_{49}$;
and, preferably, the cationic surfactants are chosen from those of formula (II) and those of formula (V), and even more preferentially from those of formula (II).

**13.** Composition according to any one of the preceding claims, **characterized in that** the cationic surfactant(s) are present in a content ranging from 0.01% to 10% by weight, more preferentially from 0.02% to 5% by weight and better still from 0.05% to 2% by weight, relative to the total weight of the composition.

**14.** Composition according to any one of the preceding claims, **characterized in that** it also comprises one or more propellants, preferably chosen from optionally halogenated volatile hydrocarbons, for example n-butane, propane, isobutane, pentane, and halogenated derivatives thereof; carbon dioxide; air; dimethyl ether; nitrogen, and mixtures thereof.

**15.** Composition according to the preceding claim, **characterized in that** the propellant(s) represent from 0,1% to 40% by weight, preferably from 1% to 30% by weight and more particularly from 3% to 20% by weight, relative to the total weight of the composition.

**16.** Composition according to any one of the preceding claims, **characterized in that** it is non-washing, i.e. its total content of anionic surfactants and of nonionic surfactants is less than or equal to 5% by weight, relative to the total weight of the composition.

**17.** Composition according to any one of the preceding claims, **characterized in that** it also comprises one or more amino silicones, in a content ranging from 0.01% to 10% by weight, preferably from 0.1% to 5% by weight and even more preferentially from 0.15% to 3% by weight, relative to the total weight of the composition

**18.** Process for styling keratin fibres, preferably the hair, comprising the application to said fibres of the composition as defined in any one of the preceding claims, this application optionally being followed by rinsing after an optional leave-on time.

**19.** Use of the composition as defined in any one of Claims 1 to 17, for shaping the hair.

**Patentansprüche**

1. Zusammensetzung, umfassend:

   - ein oder mehrere Vinylformamid/Vinylamin-Copolymere, umfassend:

     - Vinylamineinheiten der Formel (IA):

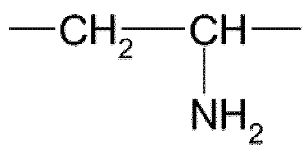
(IA)

und
- Vinylformamideinheiten der Formel (IB):

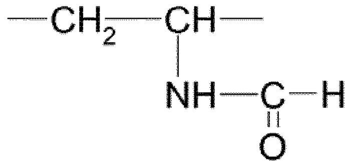
(IB);

   - ein oder mehrere fixierende Polymere, die von Vinylformamid/Vinylamin-Copolymeren verschieden sind;
   - ein oder mehrere nichtionische Tenside ausgewählt aus oxyalkylenierten Fettalkoholen, die eine lineare oder verzweigte, gesättigte oder ungesättigte Alkylkette mit mindestens 8 Kohlenstoffatomen umfassen;
   - ein oder mehrere kationische Tenside.

2. Zusammensetzung nach dem vorhergehenden Anspruch, **dadurch gekennzeichnet, dass** das bzw. die Vinylformamid/Vinylamin-Copolymer(e) 10 Mol-% bis 95 Mol-% Einheiten der Formel (IA) und 90 Mol-% bis 5 Mol-% Einheiten der Formel (IB) umfasst bzw. umfassen.

3. Zusammensetzung nach einem der beiden vorhergehenden Ansprüche, **dadurch gekennzeichnet, dass** das bzw. die Vinylformamid/Vinylamin-Copolymer(e) 10 Mol-% bis 60 Mol-% und insbesondere 20 Mol-% bis 40 Mol-% Einheiten der Formel (IA) umfasst bzw. umfassen.

4. Zusammensetzung nach einem der vorhergehenden Ansprüche, **dadurch gekennzeichnet, dass** das bzw. die Vinylformamid/Vinylamin-Copolymer(e) 30 Mol-% bis 90 Mol-% und insbesondere 60 Mol-% bis 80 Mol-% Einheiten der Formel (IB) umfasst bzw. umfassen.

5. Zusammensetzung nach einem der vorhergehenden Ansprüche, **dadurch gekennzeichnet, dass** das bzw. die Vinylformamid/Vinylamin-Copolymer(e) ausschließlich aus Einheiten der Formel (IA) und Einheiten der Formel (IB) gebildet wird bzw. werden.

6. Zusammensetzung nach einem der vorhergehenden Ansprüche, **dadurch gekennzeichnet, dass** das bzw. die Vinylformamid/Vinylamin-Copolymer(e) in Anteilen im Bereich von 0,01 Gew.-% bis 20 Gew.-%, weiter bevorzugt von 0,1 Gew.-% bis 10 Gew.-% und insbesondere von 0,15 Gew.-% bis 5 Gew.-%, bezogen auf das Gesamtgewicht der Zusammensetzung, vorliegt bzw. vorliegen.

7. Zusammensetzung nach einem der vorhergehenden Ansprüche, **dadurch gekennzeichnet, dass** die von Vinylformamid/Vinylamin-Copolymeren verschiedenen fixierenden Polymere aus nichtionischen fixierenden Polymeren und anionischen fixierenden Polymeren, vorzugsweise aus nichtionischen fixierenden Polymeren, noch weiter bevorzugt aus Vinyllactamcopolymeren wie Polyvinylpyrrolidon/-vinyllactam-Copolymeren und Polyvinylpyrrolidon/-vinylacetat-Copolymeren ausgewählt sind.

8. Zusammensetzung nach einem der vorhergehenden Ansprüche, **dadurch gekennzeichnet, dass** die von Vinylformamid/Vinylamin-Copolymeren verschiedenen fixierenden Polymere in einer Menge im Bereich von 0,1 Gew.-% bis 20 Gew.-%, vorzugsweise von 0,2 Gew.-% bis 15 Gew.-% und noch weiter bevorzugt von 0,3 Gew.-% bis 10 Gew.-%, bezogen auf das Gesamtgewicht der Zusammensetzung, vorliegen.

**9.** Zusammensetzung nach einem der vorhergehenden Ansprüche, **dadurch gekennzeichnet, dass** der bzw. die oxyalkylenierte(n) Fettalkohol(e) Fettalkohole mit 10 bis 24 Kohlenstoffatomen, vorzugsweise 12 bis 22 Kohlenstoffatomen, und 2 bis 20 Oxyalkyleneinheiten, noch besser 3 bis 10 Oxyalkyleneinheiten, am besten 3 bis 6 Oxyalkyleneinheiten, vorzugsweise Oxyethyleneinheiten, umfasst bzw. umfassen.

**10.** Zusammensetzung nach dem vorhergehenden Anspruch, **dadurch gekennzeichnet, dass** es sich bei dem oxyalkylenierten Fettalkohol um Laurylakohol mit 4 Oxyethyleneinheiten handelt.

**11.** Zusammensetzung nach einem der vorhergehenden Ansprüche, **dadurch gekennzeichnet, dass** ihr Gehalt an aus oxyalkylenierten, vorzugsweise oxyethylenierten, Fettalkoholen ausgewählten nichtionischen Tensiden 0,01 Gew.-% bis 5 Gew.-%, vorzugsweise 0,05 Gew.-% bis 2 Gew.-% und weiter bevorzugt 0,1 Gew.-% bis 1 Gew.-%, bezogen auf das Gesamtgewicht der Zusammensetzung, beträgt.

**12.** Zusammensetzung nach einem der vorhergehenden Ansprüche, **dadurch gekennzeichnet, dass** das bzw. die kationische(n) Tensid(e) ausgewählt sind aus:

- denjenigen, die der nachstehenden allgemeinen Formel (II) entsprechen:

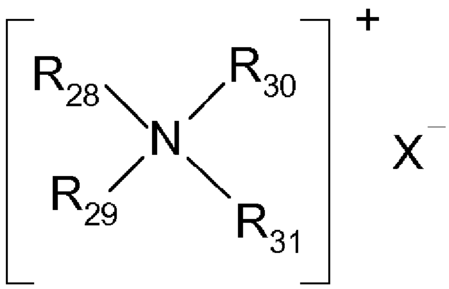

(II),

in der die Gruppen $R_{28}$ bis $R_{31}$, die gleich oder verschieden sein können, für eine lineare oder verzweigte aliphatische Gruppe mit 1 bis 30 Kohlenstoffatomen oder eine aromatische Gruppe wie Aryl oder Alkylaryl stehen, wobei mindestens eine der Gruppen $R_{28}$ bis $R_{31}$ eine Gruppe mit 8 bis 30 Kohlenstoffatomen, vorzugsweise mit 12 bis 24 Kohlenstoffatomen, bezeichnet;
- quartären Ammoniumsalzen von Imidazol, etwa denjenigen der nachstehenden Formel (III):

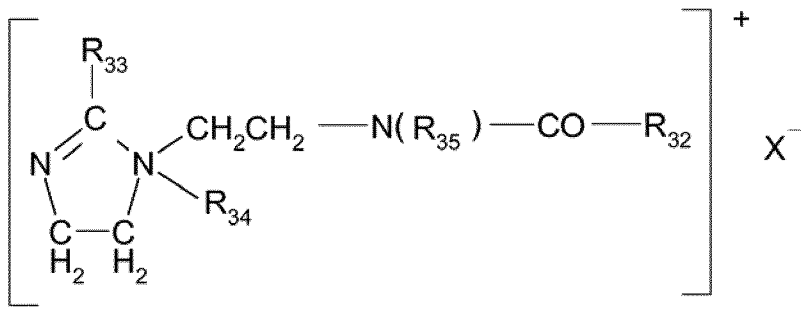

(III),

in der:

- $R_{32}$ für eine Alkenyl- oder Alkylgruppe mit 8 bis 30 Kohlenstoffatomen steht,
- $R_{33}$ für ein Wasserstoffatom, eine $C_1$- bis $C_4$-Alkylgruppe oder eine Alkenyl- oder Alkylgruppe mit 8 bis 30 Kohlenstoffatomen steht,
- $R_{34}$ für eine $C_1$- bis $C_4$-Alkylgruppe steht,
- $R_{35}$ für ein Wasserstoffatom oder eine $C_1$- bis $C_4$-Alkylgruppe steht,

- $X^-$ ein Anion ausgewählt aus der Gruppe der Halogenide, Phosphate, Acetate, Lactate, Alkylsulfate, Alkyl- oder Alkylarylsulfonate, in denen die Alkyl- und Arylgruppen vorzugsweise 1 bis 20 Kohlenstoffatome bzw. 6 bis 30 Kohlenstoffatome umfassen, ist;

- den quartären Diammonium- oder Triammoniumsalzen der Formel (IV):

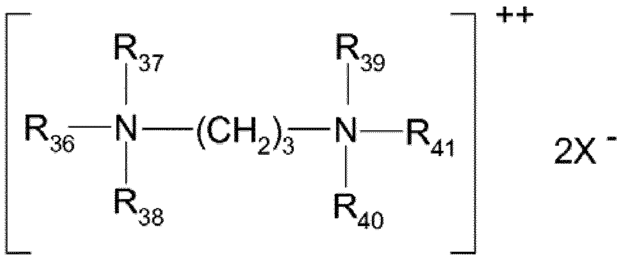

(IV),

in der:

- $R_{36}$ einen Alkylrest mit etwa 16 bis 30 Kohlenstoffatomen, der gegebenenfalls hydroxyliert und/oder durch ein oder mehrere Sauerstoffatome unterbrochen ist, bezeichnet,
- $R_{37}$ aus Wasserstoff und einem Alkylrest mit 1 bis 4 Kohlenstoffatomen oder einer Gruppe ($R_{36a}$) ($R_{37a}$) ($R_{38a}$)N-$(CH_2)_3$ ausgewählt ist,
- $R_{36a}$, $R_{37a}$, $R_{38a}$, $R_{38}$, $R_{39}$, $R_{40}$ und $R_{41}$, die gleich oder verschieden sein können, aus Wasserstoff und einem Alkylrest mit 1 bis 4 Kohlenstoffatomen ausgewählt sind und
- $X^-$ ein Anion ausgewählt aus der Gruppe der Halogenide, Acetate, Phosphate, Nitrate und Methylsulfate ist;

- quartären Ammoniumsalzen mit mindestens einer Esterfunktion wie denjenigen der nachstehenden Formel (V):

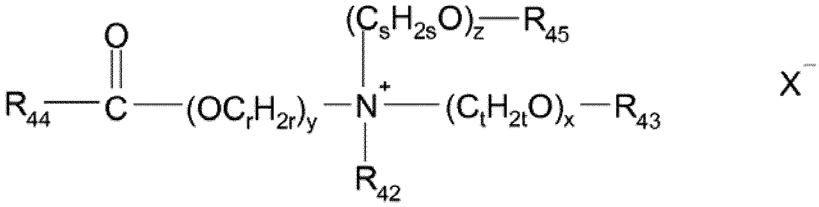

(V),

in der:

- $R_{42}$ aus $C_1$- bis $C_6$-Alkylgruppen und $C_1$- bis $C_6$-Hydroxyalkyl- oder -Dihydroxyalkylgruppen ausgewählt ist;
- $R_{43}$ ausgewählt ist aus:
- der Gruppe

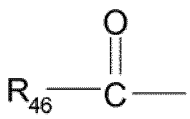

,

- Gruppen $R_{47}$, bei denen es sich um lineare oder verzweigte, gesättigte oder ungesättigte $C_1$- bis $C_{22}$-kohlenwasserstoffbasierte Gruppen handelt,
- einem Wasserstoffatom,
- $R_{45}$ ausgewählt ist aus:
- der Gruppe

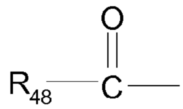
,

- Gruppen $R_{49}$, bei denen es sich um lineare oder verzweigte, gesättigte oder ungesättigte $C_1$- bis $C_6$-kohlenwasserstoffbasierte Gruppen handelt,
- einem Wasserstoffatom,
- $R_{44}$, $R_{46}$ und $R_{48}$, die gleich oder verschieden sein können, aus linearen oder verzweigten, gesättigten oder ungesättigten $C_7$- bis $C_{21}$-kohlenwasserstoffbasierten Gruppen ausgewählt sind;
- r, s und t, die gleich oder verschieden sein können, ganze Zahlen von 2 bis 6 sind;
- y eine ganze Zahl von 1 bis 10 ist;
- x and z, die gleich oder verschieden sein können, ganze Zahlen von 0 bis 10 sind;
- $X^-$ ein einfaches oder komplexes, organisches oder anorganisches Anion ist;

mit der Maßgabe, dass die Summe x + y + z gleich 1 bis 15 ist, dass dann, wenn x gleich 0 ist, $R_{43}$ $R_{47}$ bezeichnet und dass dann, wenn z gleich 0 ist, $R_{45}$ $R_{49}$ bezeichnet;

und dass die kationischen Tenside vorzugsweise aus denjenigen der Formel (II) und denjenigen der Formel (V) und noch weiter bevorzugt aus denjenigen der Formel (II) ausgewählt sind.

**13.** Zusammensetzung nach einem der vorhergehenden Ansprüche, **dadurch gekennzeichnet, dass** das bzw. die kationische(n) Tensid(e) in einem Gehalt im Bereich von 0,01 Gew.-% bis 10 Gew.-%, weiter bevorzugt von 0,02 Gew.-% bis 5 Gew.-% und noch besser von 0,05 Gew.-% bis 2 Gew.-%; bezogen auf das Gesamtgewicht der Zusammensetzung; vorliegt bzw. vorliegen.

**14.** Zusammensetzung nach einem der vorhergehenden Ansprüche, **dadurch gekennzeichnet, dass** sie ebenfalls ein oder mehrere Treibmittel umfasst, vorzugsweise ausgewählt aus gegebenenfalls halogenierten flüchtigen Kohlenwasserstoffen, zum Beispiel n-Butan, Propan, Isobutan, Pentan, und halogenierten Derivaten davon; Kohlendioxid; Luft; Dimethylether; Stickstoff und Mischungen davon.

**15.** Zusammensetzung nach dem vorhergehenden Anspruch, **dadurch gekennzeichnet, dass** das bzw. die Treibmittel 0,1 Gew.-% bis 40 Gew.-%, vorzugsweise 1 Gew.-% bis 30 Gew.-% und insbesondere 3 Gew.-% bis 20 Gew.-%, bezogen auf das Gesamtgewicht der Zusammensetzung, darstellt bzw. darstellen.

**16.** Zusammensetzung nach einem der vorhergehenden Ansprüche, **dadurch gekennzeichnet, dass** sie nichtwaschend ist, d.h. dass ihr Gesamtgehalt an anionischen Tensiden und nichtionischen Tensiden kleiner oder gleich 5 Gew.-%, bezogen auf das Gesamtgewicht der Zusammensetzung, beträgt.

**17.** Zusammensetzung nach einem der vorhergehenden Ansprüche, **dadurch gekennzeichnet, dass** sie ebenfalls ein oder mehrere Aminosilikone in einem Gehalt im Bereich von 0,01 Gew.-% bis 10 Gew.-%, vorzugsweise 0,1 Gew.-% bis 5 Gew.-% und noch weiter bevorzugt 0,15 Gew.-% bis 3 Gew.-%, bezogen auf das Gesamtgewicht der Zusammensetzung, umfasst.

**18.** Verfahren für das Frisieren von Keratinfasern, vorzugsweise dem Haar, umfassend die Anwendung der Zusammensetzung gemäß einem der vorhergehenden Ansprüche auf die Fasern, wobei auf diese Anwendung nach einer fakultativen Einwirkzeit gegebenenfalls ein Spülvorgang folgt.

**19.** Verwendung der Zusammensetzung gemäß einem der Ansprüche 1 bis 17 zur Formgebung des Haares.

## Revendications

**1.** Composition comprenant :

- un ou plusieurs copolymères vinylformamide / vinylamine comprenant :

- des motifs vinylamine de formule (IA) :

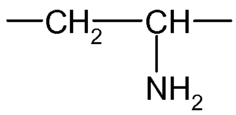

(IA),

et

- des motifs vinylformamide de formule (IB):

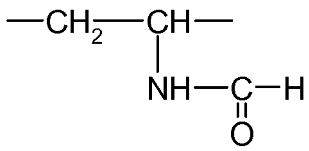

(IB) ;

- un ou plusieurs polymères fixants différents des copolymères vinylformamide / vinylamine ;
- un ou plusieurs agents tensioactifs non ioniques choisis parmi les alcools gras oxyalkylénés comprenant une chaîne alkyl linéaire ou ramifiée, saturée ou insaturée, comportant au moins 8 atomes de carbone; et
- un ou plusieurs agents tensioactifs cationiques.

2. Composition selon la revendication précédente, **caractérisée en ce que** le ou les copolymères vinylformamide / vinylamine comprennent de 10 à 95 % en moles de motifs de formule (IA) et de 90 à 5 % en moles de motifs de formule (IB).

3. Composition selon l'une quelconque des revendications précédentes, **caractérisée en ce que** le ou les copolymères vinylformamide / vinylamine comprennent de 10 à 60% en moles de motif de formule (IA) et plus particulièrement de 20 à 40% en moles.

4. Composition selon l'une quelconque des revendications précédentes, **caractérisée en ce que** le ou les copolymères vinylformamide / vinylamine comprennent de 30 à 90% en moles de motif de formule (IB) et plus particulièrement de 60 à 80% en moles.

5. Composition selon l'une quelconque des revendications précédentes, **caractérisée en ce que** le ou les copolymères vinylformamide / vinylamine sont constitués uniquement de motifs de formule (IA) et de motifs de formule (IB).

6. Composition selon l'une quelconque des revendications précédentes, **caractérisée en ce que** le ou les copolymères vinylformamide / vinylamine sont présents dans des proportions allant de 0,01 à 20 % en poids, plus préférentiellement de 0,1 à 10 % en poids et plus particulièrement de 0,15 à 5 % en poids, par rapport au poids total de la composition.

7. Composition selon l'une quelconque des revendications précédentes, **caractérisée en ce que** les polymères fixants différents des copolymères vinylformamide / vinylamine sont choisis parmi les polymères fixants non ioniques et les polymères fixants anioniques, de préférence parmi les polymères fixants non ioniques, plus préférentiellement encore parmi les copolymères de vinyllactame, tels que les copolymères poly(vinylpyrrolidone/ vinyllactame) et les copolymères poly(vinylpyrrolidone/acétate de vinyle).

8. Composition selon l'une quelconque des revendications précédentes, **caractérisée en ce que** les polymères fixants différents des copolymères vinylformamide / vinylamine sont présents en une quantité allant de 0,1 à 20 % en poids, de préférence de 0,2 à 15 % en poids, et encore plus préférentiellement de 0,3 à 10% en poids, par rapport au poids total de la composition.

9. Composition selon l'une quelconque des revendications précédentes, **caractérisée en ce que** le ou les alcools gras oxyalkylénés comprennent des alcools gras ayant 10 à 24 atomes de carbone, de préférence de 12 à 22 atomes de carbone, et de 2 à 20 unités oxyalkylène, mieux de 3 à 10 unités oxyalkylène, mieux encore de 3 à 6 unités oxyalkylène, de préférence oxyéthylène.

**10.** Composition selon la revendication précédente, **caractérisée en ce que** l'alcool gras oxyalkyléné est l'alcool laurylique à 4 unités oxyéthylène.

**11.** Composition selon l'une quelconque des revendications précédentes, **caractérisée en ce que** sa teneur en tensioactifs non ioniques choisis parmi les alcools gras oxyalkylénés, de préférence oxyéthylénés, va de 0,01 à 5% en poids, de préférence de 0,05 à 2% en poids, et plus préférentiellement de 0,1 à 1% en poids, par rapport au poids total de la composition.

**12.** Composition selon l'une quelconque des revendications précédentes, **caractérisée en ce que** le ou les agents tensioactifs cationiques sont choisis parmi :

- ceux répondant à la formule générale (II) suivante :

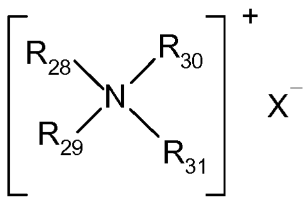

(II)

dans laquelle, les groupes $R_{28}$ à $R_{31}$, qui peuvent être identiques ou différents, représentent un groupe aliphatique, linéaire ou ramifié, comportant de 1 à 30 atomes de carbone, ou un groupe aromatique tel que aryle ou alkylaryle, au moins un des groupes $R_{28}$ à $R_{31}$ désignant un groupe comportant de 8 à 30 atomes de carbones, de préférence de 12 à 24 atomes de carbone ;

- les sels d'ammonium quaternaire de l'imidazoline, comme par exemple ceux de formule (III) suivante :

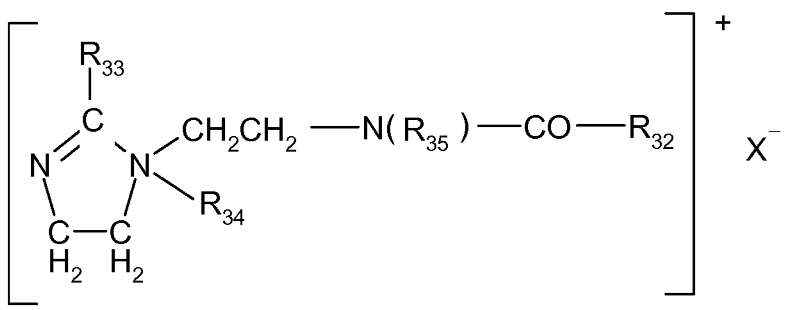

(III)

dans laquelle,

- $R_{32}$ représente un groupe alcényle ou alkyle comportant de 8 à 30 atomes de carbone,
- $R_{33}$ représente un atome d'hydrogène, un groupe alkyle en $C_1$ à $C_4$ ou un groupe alcényle ou alkyle comportant de 8 à 30 atomes de carbone,
- $R_{34}$ représente un groupe alkyle en $C_1$ à $C_4$,
- $R_{35}$ représente un atome d'hydrogène, un groupe alkyle en $C_1$ à $C_4$,
- $X^-$ est un anion choisi dans le groupe des halogénures, phosphates, acétates, lactates, alkylsulfates, alkyl- ou alkylaryl-sulfonates dont les groupements alkyl et aryl comprennent de préférence respectivement de 1 à 20 atomes de carbone et de 6 à 30 atomes de carbone ;

- les sels de di ou de triammonium quaternaire de formule (IV):

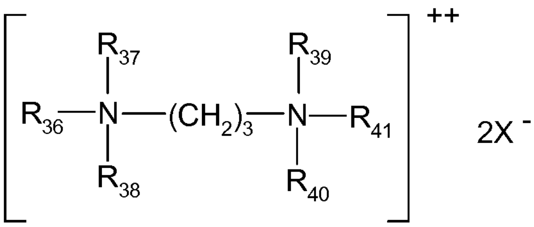

(IV)

dans laquelle,

- $R_{36}$ désigne un radical alkyle comportant environ de 16 à 30 atomes de carbone éventuellement hydroxylé et/ou interrompu par un ou plusieurs atomes d'oxygène,
- $R_{37}$ est choisi parmi l'hydrogène ou un radical alkyle comportant de 1 à 4 atomes de carbone ou un groupe $(R_{36a})(R_{37a})(R_{38a})N-(CH_2)_3$,
- $R_{36a}$, $R_{37a}$, $R_{38a}$, $R_{38}$, $R_{39}$, $R_{40}$ et $R_{41}$, identiques ou différents, sont choisis parmi l'hydrogène ou un radical alkyle comportant de 1 à 4 atomes de carbone, et
- $X^-$ est un anion choisi dans le groupe des halogénures, acétates, phosphates, nitrates et méthylsulfates ;

- les sels d'ammonium quaternaire contenant au moins une fonction ester, tels que ceux de formule (V) suivante :

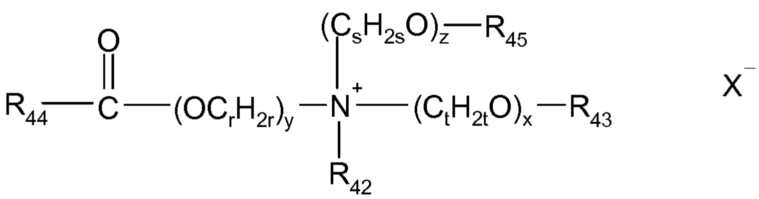

(V)

dans laquelle,

- $R_{42}$ est choisi parmi les groupes alkyles en $C_1$ à $C_6$ et les groupes hydroxyalkyles ou dihydroxyalkyles en $C_1$ à $C_6$ ;
- $R_{43}$ est choisi parmi :

  - le groupe

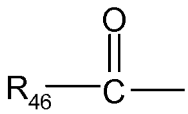

  - les groupes $R_{47}$ qui sont des groupes hydrocarbonés en $C_1$ à $C_{22}$, linéaires ou ramifiés, saturés ou insaturés,
  - l'atome d'hydrogène,

- $R_{45}$ est choisi parmi :

  - le groupe

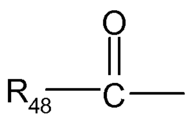

  - les groupes $R_{49}$ qui sont des groupes hydrocarbonés en $C_1$ à $C_6$, linéaires ou ramifiés, saturés ou insaturés,

- l'atome d'hydrogène,

- $R_{44}$, $R_{46}$ et $R_{48}$, identiques ou différents, sont choisis parmi les groupes hydrocarbonés en $C_7$ à $C_{21}$, linéaires ou ramifiés, saturés ou insaturés ;
- r, s et t, identiques ou différents, sont des entiers valant de 2 à 6 ;
- y est un entier valant de 1 à 10 ;
- x et z, identiques ou différents, sont des entiers valant de 0 à 10 ;
- $X^-$ est un anion simple ou complexe, organique ou inorganique ;

sous réserve que la somme x + y + z vaut de 1 à 15, que lorsque x vaut 0 alors $R_{43}$ désigne $R_{47}$ et que lorsque z vaut 0 alors $R_{45}$ désigne $R_{49}$ ;
et de préférence les agents tensioactifs cationiques sont choisis parmi ceux de formule (II) et ceux de formule (V), et encore plus préférentiellement parmi ceux de formule (II).

**13.** Composition selon l'une quelconque des revendications précédentes, **caractérisée en ce que** le ou les agents tensioactifs cationiques sont présents en une teneur allant de 0,01 à 10% en poids, plus préférentiellement de 0,02 à 5% en poids, et mieux encore de 0,05 à 2% en poids, par rapport au poids total de la composition.

**14.** Composition selon l'une quelconque des revendications précédentes, **caractérisée en ce qu'**elle comprend en outre un plusieurs agents propulseurs, de préférence choisis parmi les hydrocarbures volatiles, éventuellement halogénés, par exemples le n-butane, le propane, l'isobutane, le pentane et leurs dérivés halogénés; le dioxyde de carbone; l'air ; le diméthyléther; l'azote, et leurs mélanges.

**15.** Composition selon la revendication précédente, **caractérisée en ce que** le ou les agents propulseurs représentent de 0,1 à 40 % en poids, de préférence de 1 à 30 % en poids, et plus particulièrement de 3 à 20% en poids, par rapport au poids total de la composition.

**16.** Composition selon l'une quelconque des revendications précédentes, **caractérisée en ce qu'**elle est non lavante, c'est-à-dire que sa teneur totale en tensioactifs anioniques et en tensioactifs non ioniques est inférieure ou égale à 5% en poids, par rapport au poids total de la composition.

**17.** Composition selon l'une quelconque des revendications précédentes, **caractérisée en ce qu'**elle comprend en outre une ou plusieurs silicones aminées, en une teneur allant de 0,01% à 10% en poids, de préférence de 0,1% à 5% en poids, plus préférentiellement de 0,15% à 3% en poids, par rapport au poids total de la composition.

**18.** Procédé de coiffage des fibres kératiniques, de préférence des cheveux, comprenant l'application sur lesdites fibres de la composition telle que définie dans l'une quelconque des revendications précédentes, cette application étant suivie ou non d'un rinçage après un éventuel temps de pose.

**19.** Utilisation de la composition telle que définie dans l'une quelconque des revendications 1 à 17 pour la mise en forme des cheveux.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- FR 3002142
- EP 2098217 A
- US 4874554 A
- US 4137180 A
- FR 1222944
- DE 2330956
- LU 75370
- LU 75371
- FR 1580545
- FR 2265782
- FR 2265781
- FR 1564110
- FR 2439798
- US 2047398 A
- US 2723248 A
- US 2102113 A
- GB 839805 A
- FR 2350384
- FR 2357241
- FR 2198719
- US 4128631 A
- EP 080976 A
- FR 2077143
- FR 2393573
- US 3589578 A
- US 4031307 A
- US 4131576 A
- US 3836537 A
- FR 1400366
- EP 95238 A
- US 4185087 A
- EP 0530974 A